# EUROPEAN PATENT SPECIFICATION

(11) **EP 3 759 253 B1**
(45) Date of publication and mention of the grant of the patent: **27.03.2024**
(21) Application number: 19708648.1
(22) Date of filing: 25.02.2019
(51) Int. Cl.: C12Q 1/686

(54) **METHODS FOR DETECTING TARGET POLYNUCLEOTIDES**
VERFAHREN ZUR ERKENNUNG VON ZIELPOLYNUKLEOTIDEN
MÉTHODES POUR DÉTECTER DES POLYNUCLÉOTIDES CIBLES

(30) Priority: 26.02.2018 GB 201803019
(43) Date of publication of application: 06.01.2021
(60) Divisional application: 24157957.2
(73) Proprietor: NunaBio Limited, NE1 8BF (GB)
(72) Inventor: PIKE, Andrew, Newcastle upon Tyne Tyne and Wear NE1 7RU (GB); TUITE, Eimer, Newcastle upon Tyne Tyne and Wear NE1 7RU (GB); HEDLEY, Joseph, Newcastle upon Tyne Tyne and Wear NE1 7RU (GB); WHITFIELD, Colette, 55128 Mainz (DE); LUNN, Samantha, Newcastle upon Tyne Tyne and Wear NE1 7RU (GB); LITTLE, Rachel, Backworth Newcastle Upon Tyne NE27 0XQ (GB); BAHRA, Mantej, Leicester Leicestershire LE2 5WD (GB); IJIRO, Kuniharu, Sapporo 064-0954 (JP); MITOMO, Hideyuki, Sapporo 001-0024 (JP)
(74) Representative: Definition IP Limited
(86) International application number: PCT/GB2019/050512
(87) International publication number: WO 2019/162699

(56) References cited:
- WO-A1-95/30774
- WO-A1-2006/110680
- US-A1- 2005 191 636
- RADTKEY R ET AL: "Rapid, high fidelity analysis of simple sequence repeats on an electronically active DNA microchip", NUCLEIC ACIDS RESEARCH, INFORMATION RETRIEVAL LTD, vol. 28, no. 7, 1 April 2000 (2000-04-01), pages e17-i, XP002201637, ISSN: 0305-1048, DOI: 10.1093/NAR/28.7.E17 -& SOSNOWSKI R G ET AL: "RAPID DETERMINATION OF SINGLE BASE MISMATCH MUTATIONS IN DNA HYBRIDS BY DIRECT ELECTRIC FIELD CONTROL", PROCEEDINGS OF THE NATIONAL ACADEMY OF SCIENCES OF THE UNITED STATES OF AMERICA, NATIONAL ACADEMY OF SCIENCES, US, vol. 94, 1 February 1997 (1997-02-01), pages 1119-1123, XP000857636, ISSN: 0027-8424, DOI: 10.1073/PNAS.94.4.1119
- COLETTE J. WHITFIELD ET AL: "Enzymatic Method for the Synthesis of Long DNA Sequences with Multiple Repeat Units", ANGEWANDTE CHEMIE, INTERNATIONAL EDITION, vol. 54, no. 31, 27 July 2015 (2015-07-27) , pages 8971-8974, XP055574116, ISSN: 1433-7851, DOI: 10.1002/anie.201502971
- HEMAT F ET AL: "A Rapid and Efficient PCR-Based Method for Synthesizing High-Molecular-Weight Multimers of Oligonucleotides", BIOCHEMICAL AND BIOPHYSICAL RESEARCH COMMUNICATIONS, ELSEVIER, AMSTERDAM, NL, vol. 205, no. 1, 30 November 1994 (1994-11-30), pages 475-481, XP024765631, ISSN: 0006-291X, DOI: 10.1006/BBRC.1994.2690 [retrieved on 1994-11-30]
- ADESSI C ET AL: "Solid phase DNA amplification: characterization of primer attachment and amplification mechanisms", NUCLEIC ACIDS RESEARCH, OXFORD UNIVERSITY PRESS, GB, vol. 28, no. 20, 15 October 2000 (2000-10-15), page e87, XP002169484, ISSN: 0305-1048, DOI: 10.1093/NAR/28.20.E87

## Description

The present invention provides a thermocycling method for increasing the number of tandem repeats of a unit sequence that is 1 to 60 nucleotides long in a linear polynucleotide.

### Background

Methods for determining the presence of a target polynucleotide sequence in a test sample are used routinely in basic research, as well as having significant utility in diagnostics and therapeutics. Libraries of sequence-defined polynucleotides may also be used as identifiers (DNA bar codes) in security applications.

The presence or absence of target polynucleotide sequences (e.g. DNA or RNA) in a test sample can readily be determined using several methods known in the art. Non-limiting examples of such methods include in situ hybridisation, microarray analysis, PCR and next generation sequencing. Such methods have proven useful in, for example, identifying or monitoring biomarkers for disease or targets for therapy.

In situ hybridisation (ISH) is based on the complementary pairing of labelled DNA or RNA probes with normal or abnormal polynucleotide sequences in intact chromosomes, cells or tissue sections. Compared with other molecular biology techniques applicable to anatomical pathology, ISH enjoys better rapport with histopathologists because of its similarity to immunohistochemistry. It has the unique advantage over other molecular biology techniques, largely based on probe hybridisation with polynucleotide extracted from homogenised tissue samples, of allowing localisation and visualisation of target polynucleotide sequences within morphologically identifiable cells or cellular structures.

Polynucleotide arrays or more simply DNA arrays are a group of technologies in which specific DNA sequences are either deposited or synthesized in a 2-D (or sometimes 3-D) array on a surface in such a way that the DNA is covalently or non-covalently attached to the surface. In typical use, a DNA array is used to probe a solution of a mixture of labelled polynucleotides and the binding (by hybridization) of these "targets" to the "probes" on the array is used to measure the relative concentrations of the polynucleotide species in solution. By generalizing to a very large number of spots of DNA, an array can be used to quantify an arbitrarily large number of different polynucleotide sequences in solution. An alternative labelling strategy is to label the hybridized species once it is formed. Two advantages of this technique are that the target probe does not require labelling and that no signal is generated in the absence of the target. A disadvantage is that per surface bound probe there is only one hybridization event and thus one signalling event which overall limits the sensitivity of the technique.

The most common method of analysis for disease state DNA or RNA is the use of Polymer Chain Reaction (PCR) based assays. A biological sample will contain DNA but usually at such low concentration levels that it is difficult to detect. PCR is used to amplify the DNA, or a more specific region of DNA that harbours the sequence of interest. The PCR products are subjected to analysis by any number of different methods, which can include electrophoresis to separate the fragments through an agarose gel, based on size, with the fragment bands visualized by ethidium bromide staining and UV light. Alternatively, real-time polymerase chain reaction (Real-Time PCR), also known as quantitative polymerase chain reaction (qPCR), monitors the amplification of a targeted DNA molecule during the PCR, i.e. in real-time, and not at its end, as in conventional PCR.

Sanger sequencing, also known as the chain termination method, is a technique for DNA sequencing based upon the selective incorporation of unnatural chain-terminating dideoxynucleotides (ddNTPs) by DNA polymerase during *in vitro* DNA replication. NGS is Sanger sequencing but carried out in parallel for several samples simultaneously.

ISH, PCR and NGS are powerful techniques to elucidate DNA and RNA function and as such are excellent diagnostic tools. However, these techniques require detailed sample preparation, multistep processes and specialist instrumentation. By contrast, microarrays offer a more simplified approach to identification of DNA/RNA indicative of disease states and as such are excellent "point of care" diagnostic tools to give quick results and inform on the decision of therapy for the patient. However, microarrays typically have limited levels of sensitivity, as there is typically only one hybridisation event per surface bound probe, resulting in one signalling event per hybridisation reaction.

Whitfield C.J et al., 2015 describes an enzymatic method for the synthesis of long DNA sequences with multiple repeat units, wherein the method is in solution. Hemat and McEntee, 1994 describes similar PCR-based method for synthesizing high molecular weight multimers of oligonucleotides. Said method is performed in solution. Adessi C et al., 2000 describes a method of solid phase DNA amplification. There is a need for improves methods for determining the presence of target polynucleotide sequences in a test sample.

### Brief summary of the disclosure

The inventors have developed a novel method for enzymatic extension of an immobilised polynucleotide sequence comprising a tandem repeat. The method can be used to generate solid substrates such as microarrays with one or more immobilised polynucleotide sequences, wherein each immobilised polynucleotide sequence comprises a plurality of tandem repeats. Advantageously, these solid substrates can be used to detect the presence of a target polynucleotide sequence that is complementary to the repeat sequence with a higher level of sensitivity than other substrates known in the art, as each immobilised polynucleotide (also described herein as a surface bound "probe polynucleotide") provides multiple binding sites for the target sequence.

The invention removes a fundamental problem encountered in DNA hybridisation based detection systems of noise over signal ratio, as the signal is dependent on surface coverage which is no longer a limiting factor in this invention. In other words, it overcomes the sensitivity issues of the prior art as it improves the detection of signal over background noise. Advantageously, this may increase the accuracy of the detection, and/or may enable less sensitive (e.g. cheaper) detectors to be used in the detection method because of enhanced data acquisition.

The invention has broad application, and may find utility in a number of different target polynucleotide detection technologies. For example, the invention may be used in the context of single base-mismatch detection, SNP detection, gene sequencing technology and medical diagnostics (e.g. rapid diagnosis of colorectal cancers such as Lynch syndrome using BAT25 repeat sequence DNA probe). It may also be used in the context of molecular diagnostics, e.g. detecting biomarkers, therapy response, disease stratification and point of care applications.

The present invention is defined by the appended claims.

Throughout the description and claims of this specification, the words "comprise" and "contain" and variations of them mean "including but not limited to", and they are not intended to (and do not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

Also, as used herein, the singular terms "a", "an," and "the" include the plural reference unless the context clearly indicates otherwise. Unless otherwise indicated, polynucleotides are written left to right in 5' to 3' orientation; amino acid sequences are written left to right in amino to carboxy orientation, respectively.

Various aspects of the invention are described in further detail below.

### Brief description of the drawings

Embodiments of the invention are further described hereinafter with reference to the accompanying drawings, in which:
Figure 1 provides a schematic overview illustrating the surface functionalization steps involved to produce multiple repeat-sequence DNA via a surface confined PCR based reaction. i) immobilisation of bifunctional linker, ii) attachment of 5'-amino modified oligoseed, iii) hybridisation of oligoseed complement, iv) PCR based extension of the immobilised ds DNA to yield long DNA brushes. For sensing applications the extDNA surface needs to be denatured to provide a repeating single strand long DNA with multiple target sequences.
Figure 2 shows: A) an oligoseed functionalised silicon wafer cut to fit in a standard PCR eppendorf tube; and B) a schematic of the mechanism of surface immobilised oligoseed enzymatic extension.
Figure 3 provides a fluorescence image of surface with Picogreen applied a) dsDNA and b) extDNA. c) The bar chart highlighting the difference in fluorescence intensity between the two surfaces. d) The fluorescence image of a patterned DNA surface in which the extended dsDNA is confined to the circular islands. The data above relates to the formation of a [GATC]n sequence on a glass/siloxane/amide linker surface in a), b) and c) and a photolithographic patterned [G:C]ₙ sequence on a glass/siloxane/streptavidin-biotin linker.
Figure 4 provides A) an AFM image of ssDNA removed from the glass surface by dehybridisation of the extended [GATC] at 90°C in water. The DNA sample was combed onto a freshly cleaved mica substrate and imaged under tapping mode; and B) a height profile of a single DNA strand confirming the dimensions expected for ssDNA.
Figure 5 shows the change in gene shape caused by the deletion of the phenylalanine amino acid during the CFTR mutation¹⁴.
Figure 6 shows heat-cool cycle extension with Tgo-Pol Z3 exo- and [GCATCTTTCG (SEQ ID NO: 1) ]₂/[CGTAGAAAGC (SEQ ID NO:2)]₂ for 20 cycles; A) an agarose gel: lane 1 extension product after 20 cycles; B) a UV-Vis plot; and C) Image J analysis of extension product, which shows the intensity of the band as a percentage of the highest intensity compared to the ladder, L = DNA ladder.
Figure 7 shows A) a bar chart of the fluorescence intensity highlighting the difference between a surface modified with short oligomers compared to long extDNA for the CFTR sequence; and B) fluorescence images of each of the different surfaces.
Figure 8 shows heat-cool cycle extension with Tgo-Pol Z3 exo- and [GAAAAAAAAAAC (SEQ ID NO: 3)]₂/[CTTTTTTTTTTG (SEQ ID NO: 4)]₂ for 20 cycles; A) an agarose gel: lane 1 extension product after 20 cycles; B) a UV-Vis plot; C) Image J analysis of extension product, which shows the intensity of the band as a percentage of the highest intensity compared to the ladder, L = DNA ladder; and D) Sanger sequencing results of the DNA sequence.
Figure 9 shows an AFM image of solution extended DNA and the average height and length of the DNA strands confirming the dimensions expected for dsDNA.
Figure 10 shows A) a bar chart of the fluorescence intensity highlighting the difference between a surface modified with short oligo-seeds compared to long extDNA for the BAT25 sequence; and B) fluorescence images of each different surfaces.
Figure 11 shows an AFM image of long single stranded extDNA dehybridised from the surface for the BAT25 sequence and the average height and length of a single DNA strand confirming the dimensions expected for ssDNA.
Figure 12 shows A) an agarose gel length comparison for 1) GATC, 2) CFTR and 3) BAT25; and B) a comparison of the fluorescence intensities for the surfaces modified with each sequence highlighting the difference between short oligo-seed and extDNA.
Figure 13 shows a fluorescence intensity comparison of surfaces stained with DAPI and with PG.
Figure 14 shows A) the fluorescence intensity of extDNA and PG in TE buffer and H₂O; B) the fluorescence image of extDNA in PG/TE solution; and C) the fluorescence image of extDNA in PG/ H₂O solution.
Figure 15 shows the fluorescence intensity for various PG binding times.
Figure 16 shows the fluorescence intensity of extDNA grown on silicon dioxide surfaces.
Figure 17 shows the change in contact angle when extending the BAT25 sequence on a glass surface. For BAT25 sequence, see Table 3.
Figure 18 shows the change in fluorescence of the extended BAT25 surface with targets with different numbers of mismatches, demonstrating the sensitivity of the method to detecting small number of mismatches in a VNTR sequence. For mismatched sequences to be rehybridized with BAT25 probe see Table 5.

### Detailed description

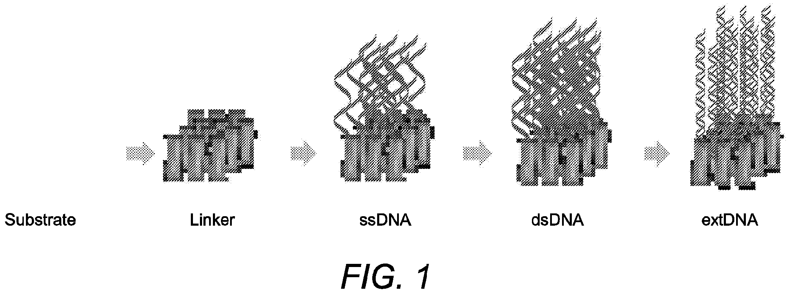

The inventors have identified a method for enzymatically extending repeat oligonucleotide sequences (oligoseeds) that are immobilised to a surface. The inventors have surprisingly shown that an immobilised oligoseed can be extended using PCR with a typical heat-cool cycle, when the oligoseed is immobilised to a solid-support. The method results in long DNA brushes directly immobilised onto a surface via a linker molecule (see Figure 1 for an overview). The subsequent denaturation to ssDNA and re-hybridisation with target complementary DNA results in increased fluorescence intensity compared to the short dsDNA on the surface. The extension increases the number of target binding sites per probe molecule hence increasing target detection.

The inventors have shown that the method is extremely versatile, as the surface, linker and DNA sequence can each be modified for the desired use. As is demonstrated in the examples section herein, the method has been successfully used to extend three different oligoseeds, employing two different linkers, and two different solid support substrates. The data presented herein clearly demonstrates the increased fluorescence signal in the extended dsDNA sample compared to the shorter dsDNA strands. It also clearly illustrates that the methodology described is suitable for integration into both optical (glass) and electronic (silicon) devices.

### Thermocycling methods

The inventors have now identified a method wherein an immobilised linear polynucleotide comprising tandem repeats of a unit sequence that is up to 60 nucleotides long can be generated using thermocycling methods. This discovery has provided new methods for generating immobilised polynucleotide probes having several binding sites for a target polynucleotide sequence, and thus has great potential in improving the sensitivity of such methods e.g. in an array format.

"Thermocycling method" refers to a method with a number of repeated cycles, wherein each cycle includes a change in temperature from a first temperature to a second (or further) temperature. Well known examples of thermocycling methods include the polymerase chain reaction (PCR).

As used herein "nucleic acid sequence", "oligonucleotide", "polynucleotide", "nucleic acid molecule" and variations thereof are used interchangeably to refer to plurality of nucleotides in either a regular or irregular sequence. Polynucleotides are typically single-stranded or double-stranded (duplex), but may adopt higher-order structures that contain three (triplex) or four (quadruplex/i-motif) strands, or may contain a mix of these configurations in different loci under suitable conditions. Polynucleotides may be short or long. They have at least two adjacent nucleotides.

The nucleotide sequence may be of genomic, synthetic or recombinant origin, and may be double-stranded or single-stranded (representing the sense or antisense strand). The term "nucleotide sequence" includes genomic DNA, cDNA, synthetic DNA, and RNA (e.g. mRNA) and analogs of the DNA or RNA generated, e.g., by the use of nucleotide analogs. In other words, modified DNA or RNA bases are also encompassed. The polynucleotide may therefore include one or a plurality of modified DNA or RNA bases. Polynucleotides bearing multiple modifications at specific sites have applications in synthetic biology, nanomaterial fabrication, bioanalytical, and sequencing applications. For example, DNA can be chemically modified at any, or all, of its three component parts - the phosphate linkage, the sugar ring, or the nucleobase. A variety of modified nucleotides can be obtained commercially as deoxynucleotidetriphosphates (dNTPs) or as phosphoramidite derivatives. These and other modified nucleotides can be synthesised and inserted into DNA or RNA either enzymatically as dNTPs, or through automated DNA synthesis as phosphoramidites.

Nucleotide residues are usually derived from the naturally occurring purine bases, namely adenine (A), guanine (G), hypoxanthine (I), and xanthine (X), and pyrimidine bases, namely cytosine (C), thymine (T), and uracil (U). Nucleotide analogues may be used at one or more of the positions within the polynucleotide sequence, such nucleotide analogues being modified in e.g. the base portion and/or the sugar portion and/or the phosphate linkage. Any nucleotide analogue can be used provided that it does not prevent the polynucleotide from hybridising and that it is accepted by polymerase as both a template and a substrate.

Nucleic acid sequences presented herein are conventionally written 5' to 3' (left to right). A "linear polynucleotide" refers to a polynucleotide which is not branched or circularised (i.e. the 3' end is not circularised with the 5' end).

In one example, the polynucleotide is DNA. In another example, the polynucleotide is RNA. The DNA or DNA may comprise natural or modified bases, including a combination thereof. Several modified bases are known in the art and suitable modified bases can therefore be readily identified by a person of skill in the art.

A thermocycling method is provided that increases the number of tandem repeats of a unit sequence that is 1 to 60 nucleotides long in a linear polynucleotide.

At the start of the thermocycling method, the linear primer polynucleotide comprises at least two copies of a unit sequence. The unit sequence is 1 to 60 nucleotides long. The linear polypeptide may comprise two or more, three or more, four or more, five or more, six or more, seven or more, eight or more, nine or more, ten or more, eleven or more, twelve or more, thirteen or more, fourteen or more, fifteen or more etc of the same unit sequence (i.e. the linear polypeptide may comprise a number of repeats of the same unit sequence). It may comprise at least 20, at least 25, at least 30, at least 35, at least 40, at least 45, at least 50, at least 55, at least 60, at least 65, at least 70, at least 75, at least 80, at least 85, at least 90, at least 95 or at least 100 copies of the unit sequence. The repeated unit sequences may be in tandem (i.e. they may be referred to as "tandem repeats"). Tandem repeats occur in polynucleotide sequences when a pattern of nucleotides (in this case a unit sequence) is repeated and the repetitions are directly adjacent to each other. By way of an example, if the unit sequence is ATTCG, a polynucleotide comprising two tandem repeats of the unit sequence would comprise the sequence ATTCGATTCG (SEQ ID NO: 5), a polynucleotide comprising three tandem repeats of the unit sequence would comprise the sequence ATTCGATTCGATTCG (SEQ ID NO: 6), a polynucleotide comprising four tandem repeats of the unit sequence would comprise the sequence ATTCGATTCGATTCGATTCG (SEQ ID NO: 7) etc. The number of tandem repeats can also be referred to as the "copy number" of the unit sequence.

The unit sequence may have any permutation of bases. Non-limiting examples of common unit sequences that may be tandemly repeated in the linear polynucleotide include: (AT)n, (GC)n, (GGC)n, (CAG)n, (GCAT)n, (GATC)n, (AAAAG)n, (AAAAAAAAAG)n (SEQ ID NO: 8). (ACTGATCAGC)n (SEQ ID NO: 9), where (xxxx) refers to the unit sequence, and n refers to the number of tandem repeats (i.e. n = the unit sequence copy number).

The unit sequence is 1 to 60 nucleotides long. The unit sequence may therefore comprise at least 1, 2, 3, 4, 5, 6, 7, 8, 9, 10, 11, 12, 13, 14, 15, 16, 17, 18, 19, 20, 21, 22,23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58 or at least 59 nucleotides (with the upper limit for each case being 60 nucleotides).

In one example, the unit sequence is a microsatellite sequence having 2 to 9 nucleotides. Alternatively, the unit sequence may be a minisatellite sequence having 10 to 60 nucleotides.

The method increases the number of tandem repeats of the unit sequence in the polynucleotide. In other words, if the starting polynucleotide (i.e. the initial immobilised primer polynucleotide) had two unit sequences (of ATTCG) in tandem (i.e. ATTCGATTCG (SEQ ID NO: 5)), the method would increase this to at least three in tandem (i.e. ATTCGATTCGATTCG (SEQ ID NO:6)). Similarly, if the starting polynucleotide had three tandem repeats of the unit sequence (i.e. ATTCGATTCGATTCG (SEQ ID NO:6)) the method would increase this to at least four repeats in tandem (i.e. ATTCGATTCGATTCGATTCG (SEQ ID NO:7)) etc.

Tandem repeats occur naturally in genomic DNA. They are designated "minisatellites" for repeats of a unit sequence that is 10-60 nucleotides long; and "microsatellites", or "short tandem repeats (STRs)", for repeats of a unit sequence that is 2-9 nucleotides long. The number of tandem repeats is designated the copy number. A variable number tandem repeat (VNTR) is a tandem repeat that varies in copy number between individuals. VNTR analysis in DNA fingerprinting is invaluable in modern forensic investigation and identity matching, as well as species typing of pathogens, fungi, and plants. Abnormalities in trinucleotide repeats are associated with genetic diseases including Huntington's disease (CAG), Friedreich's ataxia (GAA), myotonic dystrophy (CTG), and fragile X syndrome (CGG).

The method comprises the step of:
i) providing a solid substrate with a surface, the surface having immobilised thereon a plurality of distinct single stranded primer polynucleotides comprising at least two tandem repeats of a unit sequence that is 1 to 60 nucleotides long.

"Solid substrate" refers to a material or group of materials having a rigid or semi-rigid surface or surfaces. In many aspects, at least one surface of the solid substrate will be substantially flat (or planar). In other aspects, it may be desirable to physically separate regions that have different polynucleotides immobilised thereon, for example, using wells, raised regions, etched trenches, or a combination thereof. According to other aspects, the solid substrate may take the form of a bead, resin, gel, microsphere, or another geometric configuration. The substrate may therefore include a semi-solid substrate (e.g., a gel or other matrix), and/or a porous substrate (e.g., a nylon membrane or other membrane).

The surface of the solid substrate onto which a single stranded primer polynucleotide is immobilised may be composed of any suitable material, such as, but not limited to glass, polyacrylamide-coated glass, epoxy, ceramics, fused silica, silicon, quartz, various plastics, metal such as gold or silver (e.g. template striped metals such as gold or silver), nylon, gel matrix, graphene or graphene oxide. Combinations or composites of these materials are also contemplated.

The surface of the solid substrate has immobilised thereon a single stranded primer polynucleotide comprising at least two tandem repeats of a unit sequence that is 1 to 60 nucleotides long. "Primer polynucleotide" refers to the function of the polynucleotide in the thermocycling method as a primer for the polymerase chain reaction. The "primer polynucleotide" is single stranded so that it can hybridise with an at least partially complementary polynucleotide sequence, and thus initiate extension of the immobilised primer polynucleotide sequence to increase the number of tandem repeats in the immobilised sequence.

The single stranded primer polynucleotide is immobilised onto the surface of the solid substrate using any suitable immobilisation means. A linker (or any other means) may be used to immobilise the polynucleotide to the surface. Suitable surface chemistries may also be used to immobilise the polynucleotide to the surface (e.g. surface chemistries that are compatible to either ink-jet printing or spotting techniques).

In one example, the linear polynucleotide is immobilised to the surface by covalent or non-covalent bonding. The linear polynucleotide may be non-covalently immobilised to a chemically modified region of the surface.

Suitable linker/surface (or surface chemistry) combinations are well known in the art. For example, the linear polynucleotide may comprise one or more nucleotide analogues modified with functional groups that may be used for immobilisation of the polynucleotide to the surface of the solid substrate, where the functional groups may be attached to the nucleotide by flexible or rigid linkers. Exemplary functional groups include, but are not limited to, amines that react covalently with succinimidyl ester-modified labels, azides that react covalently with alkyne-modified labels, alkynes that react covalently with azide-modified labels, digoxigenin that forms a strong non-covalent interaction with anti-digoxigenin antibodies, or biotin that forms a strong non-covalent interaction with avidin or streptavidin that has been labelled with a reporter group such as a fluorescent dye non-covalently via a biotin-conjugated label or covalently on the protein. Specific examples include, but are not restricted to, 5-(3-aminoallyl)-uracil, 5- aminoallylcytosine, 5-aminoallyluracil, 7-deaza-7-propargylaminoadenine, 7-deaza-7- propargylaminoguanine, 5-propargylaminocytosine, 5-propargylaminouracil, 8-[(6-amino)hexyl-biotin]- aminoadenosine, γ-[N-(biotin-6-amino-hexanoyl)]-7-propargylamino-7-deazaadenine, γ-[N-(biotin-6-aminohexanoyl)]-5-aminoallyluracil, γ-[N-(biotin-6-amino-hexanoyl-6-aminohexanoyl)]-5-(3-aminoallyl)-uracil, digoxigenin-X-5-aminoallyl-uracil, 5-(3-azidopropyl)-uracil, 5-azido-PEG4-uracil, 5-azido-PEG4-cytosine, 5- (octa-1,7-diynyl)-uracil, 5-(octa-1,7-diynyl)-cytosine (5-C8-alkyne-C), 5-dibenzylcyclooctyl-PEG4-uracil, 5- dibenzylcyclooctyl-PEG4-cytosine, 5-trans-cyclooctene-PEG4-uracil, or any combination of these. Suitable linkers include a silane linker molecule, a biotin-streptavidin complex, a thiol-Au linker, covalent Si-C bonds to silicon, covalent Si-O bonds to silicon, covalent Si-N bonds to silicon, a nanoparticle linker, or a dynamic covalent bond.

The functional group/linker is typically attached to the 5' end of the single stranded linear polynucleotide (or at least is attached to the linear polynucleotide in a manner that enables immobilisation of the 5' end of the linear polynucleotide to the surface of the solid substrate).

The tandem repeats are typically located at the 3' terminal end of the immobilised single stranded primer polynucleotide. In some examples, the tandem repeats may constitute the last nucleotides of the linear polynucleotide (in a 5' to 3' direction).

The method further comprises the step of:
i) contacting the immobilised primer polynucleotides with a single stranded template polynucleotide comprising at least two tandem repeats that are complementary to the unit sequence of the primer polynucleotides under hybridisation conditions that permit mismatched duplex formation between a unit sequence and its complement such that a 5' overhang of the template polynucleotide is generated, wherein the 5' overhang comprises at least one tandem repeat that is complementary to the unit sequence of the primer polynucleotide.

This step is also referred to herein as the "annealing step", "hybridisation step", or variations thereof.

In this context, the "single stranded template polynucleotide comprising at least two tandem repeats that are complementary to the unit sequence of the primer polynucleotide" can also be described as a "template polynucleotide" (or "template").

The template polynucleotide comprises as least two tandem repeats that are complementary to the unit sequence of the primer polynucleotide. The term "complementary" is used in its normal context in the art. As an example, if the unit sequence of the primer polynucleotide is 5' ATCG 3', then the tandem repeat of the template polynucleotide will be 5' CGAT 3' (i.e. the template polynucleotide will comprise at least two tandem repeats, therefore will comprise the sequence 5' CGATCGAT 3'). In other words, in this example, the tandem repeats in the template and the primer are 100% complementary.

In the context of template polynucleotides with more than two tandem repeats, the invention encompasses template polynucleotide sequences wherein at least the two tandem repeats at the 3' end are 100% complementary (and the additional tandem repeats are 100% or less than 100% complementary to the corresponding tandem repeats in the primer polynucleotide. In other words, provided that the 3' end of the immobilised primer strand has at least two repeats that are complementary to the template strand, then the rest of the template strand does not have to be 100% complementary.

The immobilised primer polynucleotide is contacted with the template polynucleotide under hybridisation conditions that permit mismatched duplex formation between a unit sequence and its complement. In this context "contacting" refers to direct contact between the primer polynucleotide and the template polynucleotide, for example in an appropriate buffer and container for the subsequent thermocycling steps of the method. Suitable buffers and containers are well known and include e.g. PCR buffers and PCR Eppendorf tubes.

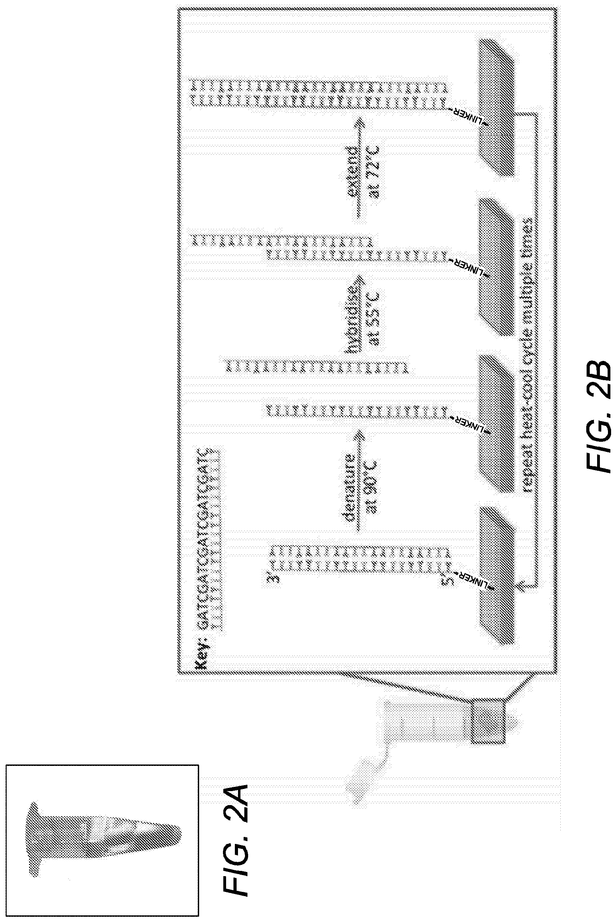

Upon contact of the primer polynucleotide with the template polynucleotide under appropriate hybridisation conditions, the complementary sequences (i.e. the unit sequence(s) of the primer polynucleotide and the complementary tandem repeats of the template polynucleotide) will hybridise to form a duplex. As each of the primer polynucleotide and the template polynucleotide have at least two tandem repeats, wherein the primer and template repeats are complementary to each other, hybridisation between the template and primer polynucleotides will, in a percentage of reactions, result in slipped alignment, wherein the resultant polynucleotide duplex does not have alignment of all of the complementary sequences (see Figure 2). In other words, mismatched duplex formation occurs (wherein "mismatched" refers to incomplete alignment between all of the complementary sequences of the unit sequence and tandem repeats in the primer polynucleotide and template polynucleotide respectively). As shown in Figure 2, a 5' overhang of the template polynucleotide may be generated, wherein the 5' overhang comprises at least one tandem repeat that is complementary to the unit sequence of the primer polynucleotide. This may then serve as a template in the thermocycling method to extend the immobilised primer polynucleotide, and thus increase the number of tandem repeats in the immobilised linear polynucleotide.

As used herein, "hybridisation conditions" refer to the reagents and reaction conditions (e.g. temperature, time etc) that are used. It describes conditions for hybridization and washing. Typically, hybridisation conditions may be stringent or moderate. The hybridisation conditions used in the context of the methods described herein permit mismatched duplex formation and therefore may be either moderate or stringent. Preferably, the hybridisation between the unit sequence of the immobilised polynucleotide and the complementary sequence of the template oligonucleotide will form a stable duplex at 65°C and below. It is preferred that a mismatched duplex may be formed at temperatures up to 65 °C, for example between 55°C and 65 °C, optionally for a time period of between 1 to 30 seconds.

Moderate and stringent conditions are known to those skilled in the art and can be found in available references (e.g., Current Protocols in Molecular Biology, John Wiley & Sons, N.Y., 1989, 6.3.1-6.3.6). Aqueous and non-aqueous methods are described in that reference and either can be used. A preferred example of stringent hybridization conditions are hybridization in 6× sodium chloride/sodium citrate (SSC) at about 45°C, followed by one or more washes in 0.2× SSC, 0.1% (w/v) SDS at 50°C. Another example of stringent hybridization conditions are hybridization in 6× SSC at about 45°C, followed by one or more washes in 0.2× SSC, 0.1% (w/v) SDS at 55°C. A further example of stringent hybridization conditions are hybridization in 6× SSC at about 45°C, followed by one or more washes in 0.2× SSC, 0.1% (w/v) SDS at 60°C. Preferably, stringent hybridization conditions are hybridization in 6× SSC at about 45°C, followed by one or more washes in 0.2× SSC, 0.1% (w/v) SDS at 65°C. Particularly preferred stringency conditions (and the conditions that should be used if the practitioner is uncertain about what conditions should be applied to determine if a molecule is within a hybridization limitation of the invention) are 0.5 molar sodium phosphate, 7% (w/v) SDS at 65°C, followed by one or more washes at 0.2× SSC, 1% (w/v) SDS at 65°C.

The method further comprises:
iii) contacting the mismatched duplexes with a thermostable 5' to 3' polymerase and nucleotides under extension conditions that permit polynucleotide extension in a 5' to 3' direction.

This step is also referred to herein as the "extension step" or variations thereof.

The term "contacting" is defined above, and applies equally in this context. It therefore refers to direct contact between the mismatched duplex and the thermostable 5' to 3' polymerase (and nucleotides), for example in an appropriate buffer and container for the subsequent thermocycling steps of the method. Suitable buffers and containers are well known and include e.g. PCR buffers and PCR Eppendorf tubes.

Several well-known thermostable 5' to 3' polymerases are available and may be used in the methods described herein. It is preferred that a polymerase is thermostable and highly stable, such that its activity is substantially retained during prolonged incubation necessary for the extension reaction. The polymerase preferably has high processivity. It is preferred that a polymerase does not display non-specific nuclease activity. A polymerase preferably has good fidelity but can also accept a range of nucleotide analogues as both templates and substrates.

A high-fidelity polymerase with efficient proof-reading activity is therefore unsuitable. Preferably, a polymerase lacks 3'→5' exonuclease activity [3'→5' exo(-)], thereby possessing lower fidelity due to absence of proof-reading function. A person skilled in the art can determine whether a particular polymerase possesses required properties as defined above. Exemplary polymerases include, but are not limited to, Tgo-Pol Z3 exo(-) [Jozwiakowski et al., 2011 Chembiochem 12: 35-37], Deep Vent exo(-) (New England Biolabs), Vent exo(-) (New England Biolabs), Pfu exo(-) (Agilent Technologies), and Taq polymerase (many suppliers). In one example, the polymerase of choice is *Thermococcus gorgonarius* family B polymerase (Tgo-Pol) enzyme variant, Z3.

Appropriate nucleotides for thermocycling reactions are well known in the art.

Contact between the mismatched duplex, polymerase and nucleotides is under extension conditions that permit polynucleotide extension in a 5' to 3' direction. As used herein, "extension conditions" refer to the reagents and reaction conditions (e.g. temperature, time etc) that are used. It describes conditions for extension of the primer polynucleotide. Appropriate extension conditions are well known in the art. Preferably, extension is performed at a temperature of between about 65°C and 75°C, optionally for a time period of between 30 to 120 seconds. Appropriate conditions may be found, for example, in Whitfield CJ, Turley AT, Tuite EM, Connolly BA, Pike AR. Enzymatic Method for the Synthesis of Long DNA Sequences with Multiple Repeat Units .Angewandte Chemie International Edition 2015, 54(31), 8971-8974.

Steps i) to iii) of the method may be repeated at least once. In order to repeat the steps, the extended duplex generated by step iii) is denatured to generate a single stranded extended immobilised polynucleotide. The single stranded polynucleotide may then act as the primer polynucleotide in repeated cycles of steps i) to iii). The number of repeated cycles can be varied to obtain different lengths of polynucleotide. The temperatures, lengths of time per step, and number of cycles control the average polynucleotide length.

The extended duplex generated by step iii) may therefore be subjected to denaturing conditions that permit dissociation of the duplex into single stranded polynucleotides. This is also referred to herein as the "melting step". Appropriate denaturing conditions are well known in the art and include subjecting the extended duplex to a temperature of about 75-100 °C, preferably about 90 to 98 °C, optionally for about 15 to 30 seconds.

The method may therefore further include the steps of:
iv) denaturing the duplex of iii) under denaturing conditions to generate a single stranded immobilised polynucleotide; and
v) repeating steps ii) to iii) at least once to increase the number of tandem repeats in the immobilised polynucleotide.

The method described thus far provides, as a starting material, a solid substrate with a surface having immobilised thereon single stranded linear primers polynucleotide comprising at least two tandem repeats of a unit sequence that is 1 to 60 nucleotides long. This starting material may be derived from a solid substrate with a surface having immobilised thereon double stranded linear primer polynucleotides comprising at last two tandem repeats of a unit sequence that is 1 to 60 nucleotides long, wherein the double stranded linear primer polynucleotides are denatured to generate the immobilised single stranded linear primer polynucleotides starting material for the methods.

Suitable denaturing conditions are described elsewhere herein.

The solid substrate with a surface having immobilised thereon a single stranded linear primer polynucleotide comprising at least two tandem repeats of a unit sequence that is 1 to 60 nucleotides long, may therefore be provided by the steps of:
a) immobilising a double stranded linear primer polynucleotide comprising at least two tandem repeats of a unit sequence that is 1 to 60 nucleotides long on the surface of the solid substrate; and
b) denaturing the double stranded linear primer polynucleotide to provide the single stranded linear primer polynucleotide.

A double stranded linear primer polynucleotide comprising at least two tandem repeats of a unit sequence that is 1 to 60 nucleotides long is also referred to herein as an "oligoseed".

The immobilised primer polynucleotide of step i) comprises at least two tandem repeats of a unit sequence. As stated above, the polynucleotide may have at least two... to at least 100 tandem repeats of the unit sequence. In one example, the immobilised primer polynucleotide comprises at least 2, at least 5, at least 10, or at least 15 tandem repeats of the unit sequence.

### Solid substrates

A solid substrate with a surface is also described with the surface having at least one linear probe polynucleotide immobilised thereon (wherein the at least one linear probe polynucleotide comprises at least two tandem repeats of a unit sequence that is 1 to 60 nucleotides long).

The solid substrate may be generated by the thermocycling method described above, wherein the initial immobilised linear primer polynucleotide is extended to increase its number of tandem repeats of the unit sequence, wherein the extended linear polynucleotide corresponds to (is referred to herein) as the linear probe polynucleotide that is immobilised on the solid substrate. Alternatively, the solid substrate may be obtained by generating the linear probe polynucleotide (e.g. in solution) and subsequently immobilising the linear probe polynucleotide to the surface of the solid substrate.

Definitions provided in respect of a linear primer polynucleotide apply equally to a linear probe polynucleotide, unless the context specifically states otherwise. Likewise, definitions provided in respect of a solid substrate (or a surface thereof) also apply to the methods described herein (which require the presence of a solid substrate), unless the context specifically states otherwise.

The linear probe polynucleotide may comprise two or more, three or more, four or more, five or more, six or more, seven or more, eight or more, nine or more, ten or more, eleven or more, twelve or more, thirteen or more, fourteen or more, fifteen or more etc of the same unit sequence (i.e. the linear polynucleotide may comprise a number of repeats of the same unit sequence). It may at least 20, at least 25, at least 30, at least 35, at least 40, at least 45, at least 50, at least 55, at least 60, at least 65, at least 70, at least 75, at least 80, at least 85, at least 90, at least 95, at least 100 copies, at least 110 copies, at least 120 copies, at least 140 copies, at least 150 copies, at least 160 copies, at least 170 copies, at least 180 copies, at least 190 copies, at least 200 copies, or at least 250 copies etc of the unit sequence.

The surface of a solid substrate may comprise:
i) a plurality of discrete spaced apart regions having a linear probe (or primer) polynucleotide immobilised thereon; and
ii) inter-regional areas between the discrete spaced apart regions, wherein the inter-regional areas are substantially free of linear probe (or primer) polynucleotides.

The linear (probe or primer) polynucleotide may be immobilised onto the surface of the substrate in a region that is referred to herein as a "discrete spaced apart region". This terminology is used to refer to a region of the surface that is distinct from other spatially separated regions of the surface which may have a different polynucleotide (or a copy of the same polynucleotide) immobilised thereon. The surface may therefore have a plurality of discrete spaced apart regions, each of which is spatially separated such that each spaced apart region can be optically separated (i.e. is optically resolvable) from neighbouring "discrete spaced apart regions" (such that any optical signal generated from one region is optically distinguishable, or discernible, from its neighbouring region). The discrete spaced apart regions are spatially separated by the provision of inter-regional areas between the discrete spaced apart regions, where the inter-regional areas are substantially free of linear probe polynucleotides. Such "inter-regional areas" are typically inert in the sense that the linear polynucleotides described herein (or other macromolecular structures) do not bind to such regions. In some examples, such inter-regional areas may be treated with blocking agents, e.g. other polymers, oxides, "chemically unreactive/incompatible sites", and the like.

The distinction between a "discrete spaced apart region" and an "inter-regional area" may be determined by the surface chemistries in these areas (wherein the surface chemistry in a discrete spaced apart region enables immobilisation of a suitable linear polynucleotide, whereas the surface chemistry of an inter-regional area does not). Alternatively, the surface chemistry may be the same for both discrete spaced apart regions and inter-regional areas, such that the distinction between them is determined by placing (immobilising) the linear polynucleotide onto the surface in certain regions (to generate a discrete spaced apart region), wherein regions without the placed (immobilised) the linear polynucleotide become "inter-regional areas".

Each discrete spaced apart region may have a defined position on the surface of the solid substrate. The required spacing between each discrete spaced apart region will depend on the methods and apparatus used for optically resolving or measuring any (direct or indirect) signal generated from the immobilised polynucleotide. It may have a size that permits immobilisation of only one linear polynucleotide described herein. Alternatively, a plurality (e.g. at least 2, at least 5, at least 10, at least 20) of identical linear probe (or primer) polynucleotides may immobilised within a single discrete spaced apart region.

Methods for determining suitable spatial separation of such regions, and determining the size of such regions, are well known in the art.

The discrete spaced apart regions may be arranged on surface in virtually any pattern in which regions have defined locations, i.e. in any regular array, which makes signal collection and data analysis functions more efficient. Such patterns include, but are not limited to, concentric circles of regions, spiral patterns, rectilinear patterns, hexagonal patterns, and the like. Preferably, regions are arranged in a rectilinear or hexagonal pattern.

The discrete spaced apart regions having the linear probe polynucleotides immobilised thereon may therefore form an array e.g. a microarray.

The surface of the solid substrate comprises a plurality of discrete spaced apart regions, wherein each discrete spaced apart region contains a distinct linear probe polynucleotide. In other words, there may be at least two distinct linear probe polynucleotides (immobilised to different discrete spaced apart regions) on the surface of the solid substrate.

As used herein, a "plurality" refers to more than one, i.e. two or more, three or more, four or more, five or more, six or more, seven or more, eight or more, nine or more, ten or more, eleven or more, twelve or more, thirteen or more, fourteen or more, fifteen or more etc. It encompasses at least 20, at least 25, at least 30, at least 35, at least 40, at least 45, at least 50, at least 55, at least 60, at least 65, at least 70, at least 75, at least 80, at least 85, at least 90, at last 95 or at least 100, at least 200, at least 300, at least 400, at least 500, at least 1000 etc.

The number of tandem repeats (and the size and sequence of a "unit sequence") in a linear polynucleotide (such as a linear probe polynucleotide; or a linear primer polynucleotide) has been discussed at length elsewhere herein. As is also described elsewhere herein, the linear polynucleotides described herein may be single stranded or double stranded DNA. Alternatively, they may be RNA or cDNA.

The unit sequence of a linear polynucleotide described herein (whether a primer or probe polynucleotide) may comprise any sequence that is desired e.g. any diagnostically relevant nucleotide sequence.

Relevant, non-limiting examples include sequences that are diagnostic of disease e.g. microsatellite instabilities (MSIs) such as BAT25, and single or multiple base mutations, as occur in, for example, the CFTR gene in cystic fibrosis. Accordingly, relevant unit sequences may be 5' GCAT**CTT**TCG 3' (SEQ ID NO: 1) (derived from CFTR Gene in Cystic Fibrosis; generated from a three-base frame shift mutation of the CFTR gene), 5' AGA TAC ATT GAC CTT '3 (SEQ ID NO: 10) (derived from the CYP450 liver enzyme, CYP29C *2/*3 which affects metabolism of Warfarin; representing a single base mutation), or 5' GAG GAC CGT GTT CAA '3 (SEQ ID NO: 11), or many others (including sequences that are the complement of the recited sequences above). Sequences of interest may readily be identified by a person of skill in the art, wherein the gene (mutation) of interest (or it's complement) is included within the sequence. Examples of appropriate sequences for warfarin genetic analysis can be found in Johnson J, Caudle K, Gong L, Whirl-Carrillo M, Stein C, Scott S, et al. Clinical Pharmacogenetics Implementation Consortium (CPIC) Guideline for Pharmacogenetics-Guided Warfarin Dosing: 2017 Update. Clin Pharmacol Ther. 2017 Sep 1;102(3):397-404; Stubbins MJ, Harries LW, Smith G, Tarbit MH, Wolf CR. Genetic analysis of the human cytochrome P450 CYP2C9 locus. Pharmacogenetics. 1996;6(5):429-39; Rettie AE, Wienkers LC, Gonzalez FJ, Trager WF, Korzekwa KR. Impaired (S)-warfarin metabolism catalysed by the R144C allelic variant of CYP2C9. Pharmacogenetics. 1994 Feb;4(1):39-42; Steward DJ, Haining RL, Henne KR, Davis G, Rushmore TH, Trager WF, et al. Genetic association between sensitivity to warfarin and expression of CYP2C9*3. Vol. 7, Pharmacogenetics. 1997. p. 361-7; and Lee CR, Goldstein JA, Pieper JA. Cytochrome P450 2C9 polymorphisms: a comprehensive review of the in-vitro and human data. Pharmacogenetics. 2002 Apr;12(3):251-63.

### Methods for determining the presence of a linear target sequence in a test sample

The solid substrates described herein may be used to determine whether (or not) a linear target sequence of interest is present within a test sample. The solid substrate has a surface having a linear probe polynucleotide(s) immobilised thereon. The linear probe polynucleotide comprises at least two tandem repeats of a unit sequence. Each unit sequence may act as a probe (in other words, as a binding site) for a complementary linear target sequence of interest when at least a portion of each unit sequence comprises a nucleic acid sequence that is complementary to the linear target sequence of interest. The solid substrates described herein therefore provide a detection technology with improved sensitivity for detecting target polynucleotide sequences in a test sample, as each immobilised probe polynucleotide comprises several target binding sites.

Described herein but not part of the claimed invention is a method for determining the presence of a linear target polynucleotide sequence in a test sample is therefore provided, comprising the steps of:
i) providing a solid substrate as described elsewhere herein, wherein the unit sequence of the immobilised linear probe polynucleotide comprises a nucleic acid sequence that is complementary to the sequence of a linear target polynucleotide sequence of interest;
ii) contacting a test sample with the immobilised linear probe polynucleotide under conditions that permit duplex formation between the linear target polynucleotide sequence and the complementary portion of the unit sequence of the immobilised linear probe polynucleotide; and
iii) detecting duplex formation, wherein duplex formation indicates that the target polynucleotide sequence in present within the test sample.

The linear target polynucleotide may be any polynucleotide sequence of interest. The linear target polynucleotide sequence must be capable of hybridising with the complementary portion of the unit sequence of the linear probe polynucleotide, therefore an appropriate linear probe polynucleotide must be immobilised onto the solid substrate. For example, if the linear target polynucleotide has the sequence 5' ATCGAA 3', then the unit sequence of the linear probe polynucleotide should comprise the sequence 5' TTCGAT 3'. Appropriate unit sequences for linear target polynucleotide are readily identifiable by a person of ordinary skill in the art.

The unit sequence of the immobilised linear probe polynucleotide therefore comprises a nucleic acid sequence that is complementary to the sequence of a linear target polynucleotide sequence of interest. In this example, the unit sequence can also include other (additional) nucleic acids that are not complementary to the sequence of the linear target polynucleotide of interest. In this example, the additional nucleic acids in the unit sequence may act as a "spacer" between target binding sites in the immobilised linear probe polynucleotide.

In another example, the unit sequence consists of a nucleic acid sequence that is complementary to the sequence of a linear target polynucleotide sequence of interest (i.e. the unit sequence does not include "additional nucleic acids", in contrast to the example above).

The linear target polynucleotide may be part of a longer polynucleotide molecule in the test sample. As used herein, "linear target polynucleotide" therefore does not limit the total length (or sequence) of the polynucleotide that forms a duplex with the immobilised linear probe polynucleotide, but only refers to the sequence that is capable of hybridising to the corresponding sequence in the unit sequence of the immobilised linear probe polynucleotide (and thus the sequence in the test sample that is of interest, and/or which is informative (e.g. diagnostic/prognostic)). This may therefore be a part of a longer sequence in the test sample.

A test sample may be any appropriate sample that may contain the linear target polynucleotide of interest. A "test sample" usually means a quantity of material from a biological, environmental, medical, or patient source in which detection or measurement of a linear target polynucleotide of interest is sought. On the one hand it is meant to include a specimen or culture (e.g., microbiological cultures). On the other hand, it is meant to include both biological and environmental samples. A sample may include a specimen of synthetic origin. Biological samples may be animal, including human, fluid, solid (e.g., stool) or tissue, as well as liquid and solid food and feed products and ingredients such as dairy items, vegetables, meat and meat by-products, and waste. Biological samples may include materials taken from a patient including, but not limited to cultures, blood, saliva, cerebral spinal fluid, pleural fluid, milk, lymph, sputum, semen, needle aspirates, and the like. Biological samples may be obtained from all of the various families of domestic animals, as well as feral or wild animals, including, but not limited to, such animals as ungulates, bear, fish, rodents, etc. Environmental samples include environmental material such as surface matter, soil, water and industrial samples, as well as samples obtained from food and dairy processing instruments, apparatus, equipment, utensils, disposable and non-disposable items. These examples are not to be construed as limiting the sample types applicable to the present invention.

Several standard methods are known for detecting duplex formation. These include use of a fluorescent intercalator such as Picogreen, DAPI or sybergreen, fluorescent tagged DNA, fluorescein, redox tagged DNA, ferrocene, nanoparticles or magnetically tagged DNA. Such standard methods are discussed, for example in Comparison of DNA detection methods using nanoparticles and silver enhancement; B. Foultier ; L. Moreno-Hagelsieb ; D. Flandre ; J. Remacle Volume 152, Issue 1, IEE Proceedings - Nanobiotechnology; February 2005, p. 3 - 12; and DNA Biosensors- A review by Kavita V, J Bioengineer & Biomedical Sci 2017, 7:2; or Mikkelsen, S. R. (1996), Electrochecmical biosensors for DNA sequence detection. Electroanalysis, 8: 15-19.

Detection of duplex formation indicates that the target polynucleotide sequence in present within the test sample.

### Methods for determining the presence of a target nucleotide binding molecule in a test sample

The solid substrates described herein may also be used to determine whether (or not) a target nucleotide binding molecule of interest is present within a test sample. The solid substrate has a surface having a linear probe polynucleotide(s) immobilised thereon. The linear probe polynucleotide comprises at least two tandem repeats of a unit sequence. Each unit sequence may act as a probe (in other words, as a binding site) for a target nucleotide binding molecule of interest when at least a portion of each unit sequence comprises a nucleic acid sequence that acts as a binding sequence for the target nucleotide binding molecule of interest. The target nucleotide binding molecule of interest may be any molecule (e.g. a protein) that binds to a specific nucleotide sequence (that may be represented by a unit sequence as described herein). The solid substrates described herein therefore provide a detection technology with improved sensitivity for detecting target nucleotide binding molecules of interest in a test sample, as each immobilised probe polynucleotide comprises several target binding sites.

A method for determining the presence of a nucleotide binding molecule in a test sample is therefore descibed, comprising the steps of:
i) providing a solid substrate as described elsewhere herein, wherein the unit sequence of the immobilised linear probe polynucleotide comprises a nucleic acid sequence that is a binding site for a nucleotide binding molecule of interest;
ii) contacting a test sample with the immobilised linear probe polynucleotide under conditions that permit binding between the nucleotide binding molecule and the portion of the unit sequence of the immobilised linear probe polynucleotide which acts as it's binding site; and
iii) detecting binding between the immobilised linear probe polynucleotide and the nucleotide binding molecule, wherein binding indicates that the nucleotide binding molecule in present within the test sample.

The nucleotide binding molecule may be any molecule of interest that is capable of binding to the appropriate portion of the unit sequence of the linear probe polynucleotide. Non-limiting examples include transcription factors, DNA repair proteins or histones. An appropriate linear probe polynucleotide must be immobilised onto the solid substrate. For example, if the nucleotide binding molecule binds to the sequence 5' ATCGAA 3', then the unit sequence of the linear probe polynucleotide should comprise the 5' ATCGAA 3'. Appropriate unit sequences are readily identifiable by a person of ordinary skill in the art.

A test sample may be any appropriate sample that may contain the nucleotide binding molecule of interest. A "test sample" usually means a quantity of material from a biological, environmental, medical, or patient source in which detection or measurement of a nucleotide binding molecule of interest is sought. On the one hand it is meant to include a specimen or culture (e.g., microbiological cultures). On the other hand, it is meant to include both biological and environmental samples. A sample may include a specimen of synthetic origin. Biological samples may be animal, including human, fluid, solid (e.g., stool) or tissue, as well as liquid and solid food and feed products and ingredients such as dairy items, vegetables, meat and meat by-products, and waste. Biological samples may include materials taken from a patient including, but not limited to cultures, blood, saliva, cerebral spinal fluid, pleural fluid, milk, lymph, sputum, semen, needle aspirates, and the like. Biological samples may be obtained from all of the various families of domestic animals, as well as feral or wild animals, including, but not limited to, such animals as ungulates, bear, fish, rodents, etc. Environmental samples include environmental material such as surface matter, soil, water and industrial samples, as well as samples obtained from food and dairy processing instruments, apparatus, equipment, utensils, disposable and non-disposable items. These examples are not to be construed as limiting the sample types applicable to the present invention.

Any of the standard methods known in the art for detecting binding between a nucleotide binding molecule and a linear probe polynucleotide may be used in the context of the invention [for example, Crystal structure of Δ-[Ru(bpy)2dppz]2+ bound to mismatched DNA reveals side-by-side metalloinsertion and intercalation Nature Chemistry, 2012 Volume 4, No 8, 615 - 620; Hang Song, Jens T. Kaiser & Jacqueline K. Barton; or Label-free detection of DNA-binding proteins based on microfluidic solid-state molecular beacon sensor.

Anal Chem. 2011, 83(9), 3528-32. Wang J, Onoshima D, Aki M, Okamoto Y, Kaji N, Tokeshi M, Baba Y.; or Annu Rev Anal Chem, 2011, 4(1), 105-128. Metal Ion Sensors Based on DNAzymes and Related DNA Molecules; Xiao-Bing Zhang, Rong-Mei Kong, and Yi Lu.

The comparison of sequences and determination of percent identity or complementarity between two sequences can be accomplished using a mathematical algorithm. In a preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used if the practitioner is uncertain about what parameters should be applied to determine if a molecule is within a sequence identity or homology limitation of the invention) are a BLOSUM 62 scoring matrix with a gap penalty of 12, a gap extend penalty of 4, and a frameshift gap penalty of 5.

Alternatively, the percent identity between two nucleotide sequences can be determined using the algorithm of Meyers et al. (1989) CABIOS 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-410). BLAST nucleotide searches can be performed with the NBLAST program, score = 100, wordlength = 12 to obtain nucleotide sequences homologous to nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, gapped BLAST can be utilized as described in Altschul et al. (1997, Nucl. Acids Res. 25:3389-3402). When using BLAST and gapped BLAST programs, the default parameters of the respective programs (e.g., XBLAST and NBLAST) can be used. See <http://www.ncbi.nlm.nih.gov>.

Aspects of the invention are demonstrated by the following non-limiting examples.

### EXAMPLES

### 1. Covalent immobilisation of ssDNA on a solid glass surface via a APEGDMES linker

A microscope glass slide was initially cleaned with acetone, IPA and nanopure water followed by treatment with O₂ plasma to remove any residual organic contaminants and to activate the surface with OH groups⁸. The surfaces was then modified with an acetal protected aldehyde terminated siloxane linker, APEGDMES (SEQ ID NO: 12), (acetalpolyethyleneglycoldimethylethoxysilane) by heating in toluene at 80 °C overnight. The APEGDMES modification affords a terminal acetal-protected aldehyde surface, which was simply removed in 10 % acetic acid water solution in order to present the aldehyde functionality at the top terminus of the linker. The amino modified oligoseed, 5'-NH₂-[GATC]₅-3', was covalently coupled to the aldehyde surface using sodium cyanoborohydride in order to facilitate the reductive amination reaction between the aldehyde linker and the amino functionality on the DNA strand. The oligoseed functionalised surface was then washed in nanopure water for 30 mins and 0.5 × PBS buffer to remove any physisorbed DNA strands. The short ssDNA was therefore covalently tethered to the surface.

### 2. Generation of an immobilised oligoseed and PCR extension thereof

The covalently tethered short ssDNA generated in Example 1 was then hybridised with its complementary strand 5'-[CTAG]₅-3' to form the starting 20-base oligoseed duplex necessary for the PCR-based enzymatic extension reaction. The dsDNA surface was rinsed further in nanopure water and 0.5 × PBS buffer. PCR-based heat-cool extension cycles were then performed on the DNA strand using the *Thermococcus gorgonarius* family B polymerase (Tgo-Pol) enzyme variant, Z3 as previously reported (Pike et al, Angew 2015). The procedure was performed as for the reported solution-based heat-cool method, however here the oligoseed modified silicon chips were cut to 0.5 cm² to fit into a standard PCR eppendorf tube for thermocycling using a heat-cool block (see Figure 2A). The total volume of reagents, Z3-enzyme, dNTPs and buffer was 180 µL, sufficient to ensure that the surface of the silicon was immersed at all times. Initially the short dsDNA was dehybridised by heating to 95 °C before cooling to 55 °C for re-hybridisation. However perfectly matched duplexes will not always form due to the repeat CATG/GTAC sequence and this mismatch is exploited to extend the DNA from off the surface. Upon re-hybridisation the complementary strand can shift by one, two or three units, (assuming an 8-base, 2 unit repeat, duplex is the mimimum required to form a stable double strand) which produces 5'-overhangs suitable for DNA polymerase extension. DNA extension then takes place at 72 °C where the enzyme adds in the matching NTP to the surface bound sequence thus increasing the length of the DNA duplex by the number of slipped repeat-units. The increase in the immoblised DNA length occurs in the z-direction away from the surface and maintains the surface packing density of the shorter oligoseeds. The heat-cool method is repeated for up to 20 cycles in order to achieve DNA brushes of around 700 bases in length (Figure 2).

### 3. Visualisation of the extended polynucleotide sequences

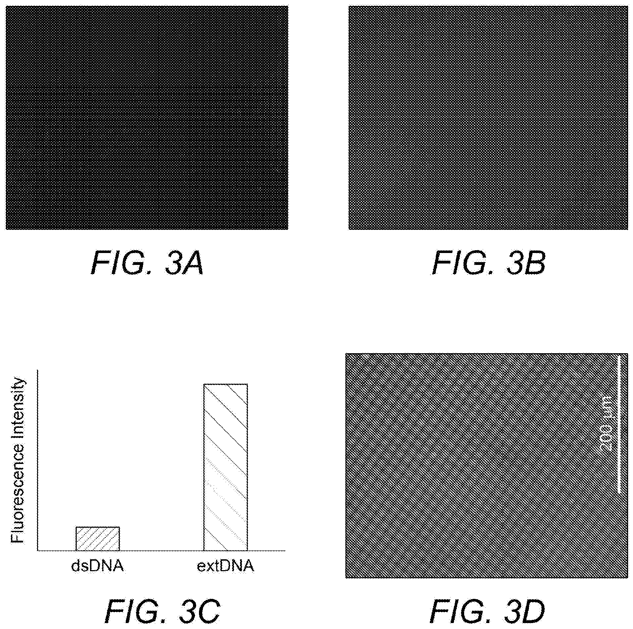

Visualisation of the increased number of DNA bases packed within the same surface area was demonstrated by the addition of the fluorescent dye Picogreen (PG) which intercalates into dsDNA. PG exhibits an >1000 fold increase in fluorescence when binding to dsDNA. After incubation in a 200 fold dilution of stock solution for 30 min, the enzymatically treated surface showed an increased fluorescence intensity after 20 heat-cool cycles compared to the untreated starting oligoseed strands on the surface, as shown in Figure 3 a) and b).

The change in fluorescence, although noticeable by eye (Figure 3a and 3b) is clearer from an integrated density analysis performed using imaged software on the two Picogreen labelled surfaces (see Figure 3c). The extension reaction dramatically increases the number of Picogreen binding sites per point of surface attachment, hence increasing the fluorescence intensity. There was a clear distinction between the short oligo seed and the extDNA surface attributed to the increased number of PG binding sites per probe molecule.

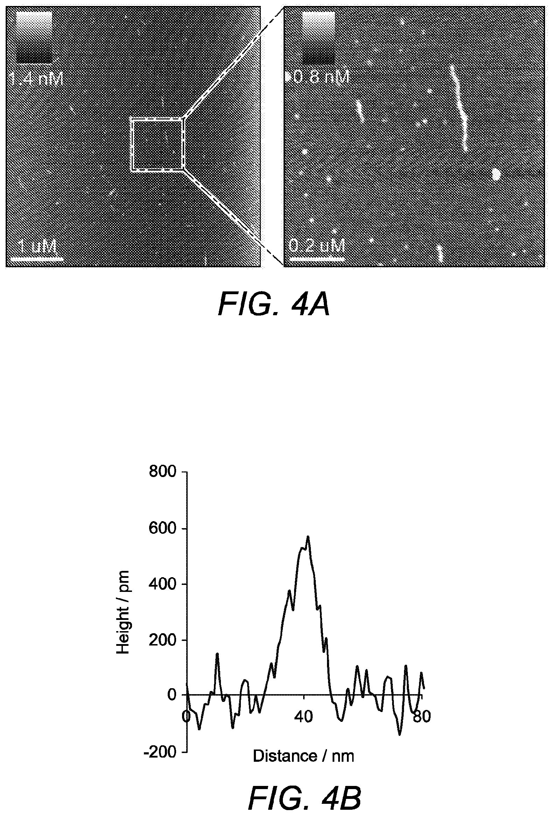

The increase in fluorescence intensity was an excellent indicator that the DNA has extended off the surface. In order to further confirm the increase in DNA length, the extDNA on the surface was dehybridised and the long complementary strand, which was not covalently attached to the surface, was removed from the surface and collected. The extDNA surface was immersed twice in nanopure water heated to 95 °C for dehybridisation. AFM was used to visualise the long DNA by taking 5 µL of sample and placing it on freshly cleaved mica via molecular combing which is known to extend the flexible DNA strand.¹⁰ The AFM image for the ssDNA can be seen in Figure 4a. Many of the ssDNA strands were aggregated on the surface which is typical for ssDNA,¹¹ however some single strands are visible with an average height of 0.5 nm (see Figure 4b), comparable to previously reported dimensions for ssDNA.¹² Analysis of the average length of the ssDNA strands gave a range from 160-300 bp confirming the successful extension of the oligo seed off the surface. The length of the strands obtained from the surface was shorter than observed in the agarose gel from the solution-based extension. Without wishing to be bound to any particular theory, this could be due to the steric hindrance of the surface restricting full movement of the enzyme hence leading to a reduced reaction rate.

### 4. CTFR gene sequence detection

Long DNA brushes with repeat sequences could distinguish between single base mismatches in diseases, ideal for DNA biosensing. Many diseases are the result of mutations in the gene causing differences in the DNA sequence.¹⁴ A platform to differentiate between the specific sequences allows for early detection of disease or determination of the type of defect.

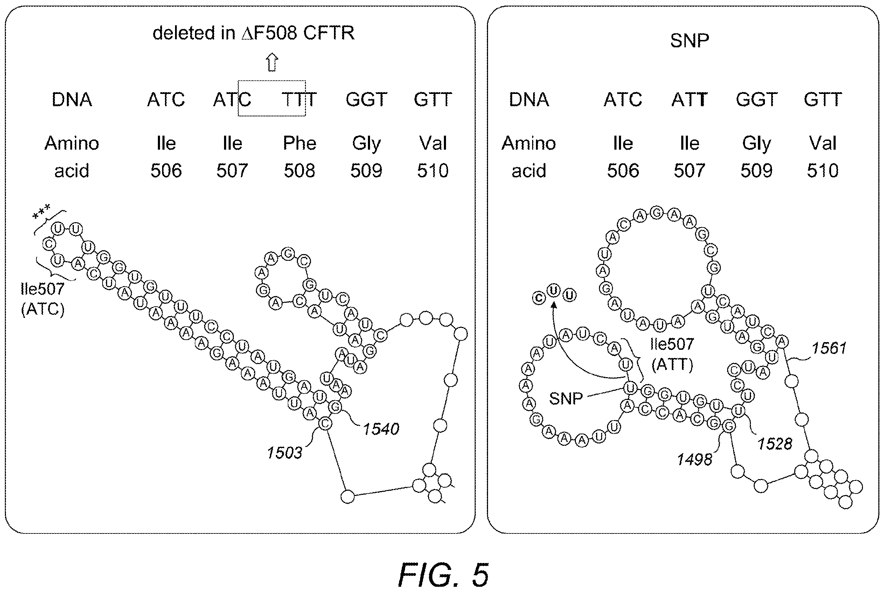

One, non-limiting, example of a disease where this type of device would be especially beneficial is the cystic fibrosis transmembrane conductance regulator known as the CFTR gene. The CFTR gene codes for a protein and this acts as a channel to control the transport of chloride ions in and out of cells to manage water content for mucus production. The protein is also necessary for sodium regulation in the lungs and pancreas. The most common frameshift mutation seen in CFTR is a 3-base deletion of the bases CTT, known as delta F508, resulting in a change in gene coding by the removal of the amino acid phenylalanine (see Figure 5). The deletion of amino acid results in the complete distortion of the gene shape which causes the break-down of the ion channel. Without a functioning ion channel, cells which line the pancreas, lungs and other organs generate thick mucus resulting in the blockage of airways and glands. Detecting this change early would allow immediate treatment, reducing the effects of the disease to improve the quality of life.

Therefore, the DNA sequence for the CFTR delta F508 mutation was chosen as the next sequence to trial with the extension method off the surface.

The CFTR sequence was initially extended in solution as described herein to ensure this specific sequence is extendable using this method. The duplex was made from the amino-modified probe and target DNA sequences described in table 1.

**Table 1. The amino-modified DNA sequences used for the CFTR extension.**

| Name | Sequence (X = amino-c6) |
|---|---|
| CFTR-probe | 5' - X - GCATCTTTCGGCATCTTTCG - 3' (SEQ ID NO: 13) |
| CFTR-target | 5' - CGAAAGATGCCGAAAGATGC - 3' (SEQ ID NO: 14) |

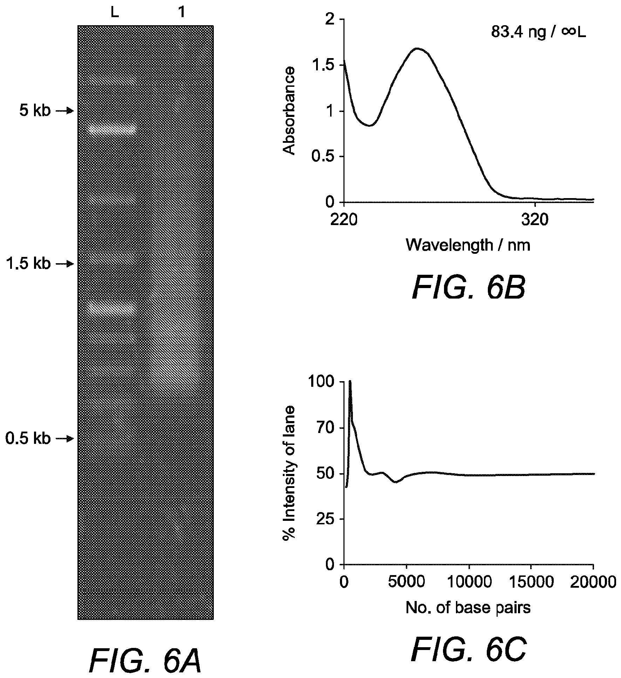

DNA products were analysed by agarose gel electrophoresis (Figure 6a) and the modal length of 300 bp was determined using imaged analysis software. The length of this DNA sequence was lower than for the GATC sequence and also exhibited a lower concentration. However, it was extending to a length which gives 30 repeat sequences per probe strand, therefore the extension off the surface was still undertaken.

Having established that both the extension of an oligoseed immobilised on a surface is possible and that the extended sequence exhibits an increased fluorescent signal, a glass slide was prepared with an oligoseed of sequence, 5' GCAT**CTT**TCG 3' (SEQ ID NO: 1), the CTFR gene which after mutation results in a three base mismatched frame-shift (bold), 5' GCATTCGAGC 3' (SEQ ID NO: 15). Clearly it would be diagnostically important if this shift in base sequence could be rapidly analyzed to aid in the early detection of Cystic Fibrosis.

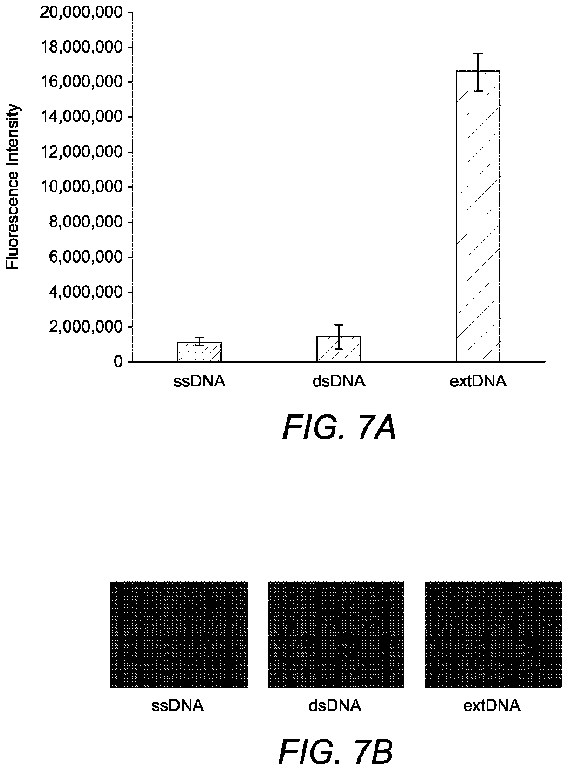

The CFTR oligo-seed sequence was immobilised on a glass surface and subjected to the reaction conditions for enzymatic extension for 20 cycles. After addition of PG to the surfaces, fluorescence images were obtained to observe the increase in fluorescence intensity for the extDNA sample (see Figure 7). The extDNA exhibited increased fluorescence intensity compared to the short oligo seeds however, the difference was not as considerable as for the GATC sequence. Without wishing to be bound to any particular theory, this is could be a consequence of the fact that the CFTR sequence in solution did not extend as well as the GATC sequence therefore there was less DNA bp for the PG to bind to, which could have resulted in a decreased fluorescence intensity compared to the GATC surface.

The contact angle of the each of the surfaces, ssDNA, dsDNA and extDNA, was determined in order to examine the hydrophobicity of the different surface modifications (see Table 2). The contact angle for dsDNA was 32.33°. After the extension from the surface, the contact angle increased dramatically to 73.11°. Without wishing to be bound to any particular theory, the extensive increase in hydrophobicity can be attributed to the hydrophobic nature of the DNA bases. In the duplex conformation of short oligomers the bases are sheltered from any water molecules however with long DNA, the structure is less rigid and therefore more bases could be in contact with the water droplet resulting in a hydrophobic terminal monolayer and an increase contact angle.¹⁵

**Table 2. Contact angles of a water droplet on the ssDNA, dsDNA and extDNA surface for the CFTR sequence.**

| Surface | Contact angle /° |
|---|---|
| ssDNA | 37.10 |
| dsDNA | 32.33 |
| extDNA | 73.11 |

The approach described here is able to enhance the fluorescence response by providing an elongated and densely packed array of the target gene and holds promise for the early detection of single and small number base mismatches.

### 5. Covalent immobilisation and extension of ssDNA on a silicon surface

A silicon surface, 25 mm² n-type Si<111> wafer was cleaned and modified as for the glass slide described above. Another diagnostically important oligoseed sequence consisting of a long run of ten T-bases, 5'-[GTTTTTTTTTTC]₂-3' (SEQ ID NO: 16) was immobilised via similar siloxane chemistry and then enzymatically extended to yield multiple repeats. This time the target sequence is important due to single base mis-incorporation which occur when the run of T's is either elongated or reduced during mistakes in transcription. This is an example of a potential MSI target, involved in the onset of colorectal cancers [see for example Arq Bras Cir Dig. 2012 Oct-Dec;25(4):240-4. Microsatellite instability--MSI markers (BAT26, BAT25, D2S123, D5S346, D17S250) in rectal cancer. Losso GM1, Moraes Rda S, Gentili AC, Messias-Reason IT].

The fluorescence data of this extended surface exhibited the same amplified response to the glass surface.

### 6. Covalent immobilisation and extension of sequence [G₁₀:C₁₀] from a glass surface

A glass substrate was patterned using a biotin/streptavidin substrate interface so that the target ssDNA is confined to small islands on the substrate surface. [see for example Nakamura S, Mitomo H, Aizawa M, Tani T, Matsuo Y, Niikura K, Pike AR, Naya N, Shishido A, Ijiro K. DNA Brush-Directed Vertical Alignment of Extensive Gold Nanorod Arrays with Controlled Density. ACS Omega, 2017, 2 (5), 2208-2213]. This demonstrates that the processivity of the Z3 enzyme is not hampered by the type of substrate or the more complex functionalisation approach involving a protein, streptavidin, within the linker layer. After hybridisation and extension performed as described previously, SYBR green dye was then applied to the surface. The fluorescence image in Figure 3d shows that the areas of DNA exhibit fluorescence whereas the bare tracks in between are unresponsive. This is a direct demonstration for the potential to multiplex the approach where every DNA spot is a different sequence. In order to confirm that the DNA has increased in length whilst tethered to the surface, the final dsDNA on the surface was dehybridised by heating the surface to 95 °C in water (1 mL × 2). This complementary strand which is not bound to the surface is therefore removed from the surface in the two heated washes. AFM was used to confirm the presence of long ssDNA by taking a 5 µL sample from the combined washes and spotting onto freshly cleaved mica via molecular combing in an attempt to extend the flexible molecule. The ssDNA strands appear to be aggregating together which is typical for ssDNA on a mica surface, however some single strands are observed with an average height of 0.5 nm, similar to previous dimensions as reported for ssDNA. Length analysis was performed on the single strands giving a range from 160-300 bp confirming successful extension off the surface. The extension of [GATC]₅/[CTAG]₅ in solution results in an average length of 750 bp as observed by agarose gel electrophoresis and AFM.

Interestingly, contact angle measurements exhibited an increase from 25.6° for the initial oligseed dsDNA surface to 74.8° for the enzymatic exended dsDNA surface.

### 7. Extension of Bat25 sequence

Microsatellite instabilities, MSI, are markers for genetic instability found in the majority of tumours in patients with hereditary colorectal cancer and in several sporadic colorectal cancers.¹⁶ MSI's are noncoding mononucleotide repeat sequences which display differences in allele length in tumour cells compared to normal DNA alleles from the same patient, either due to deletions or insertions. One of the most commonly used mononucleotide repeat marker used for MSI identification is the BAT25 sequence, a polyT repeat unit. The BAT25 sequence can be used without comparison to normal DNA and involves significant base deletions in virtually all tumours displaying MSI.¹⁶ Understanding the type of MSI allows for identification of tumour type and can anticipate the patient's chemotherapeutic response. There exists a need for a quick, sensitive and reproducible method for identification of the MSI to enable rapid diagnosis and treatment.

Current methods for MSI recognition involves using a specially designed panel, the Bethesda panel, in which 5 microsatellite markers are screened; the mononucleotide repeat markers, BAT25 and BAT26, and dinucleotide repeat markers, D2S123, D5S346 and D17S250. If 2-5 of the markers are mutated, the patient is deemed to have high microsatellite instabilities, MSI-H. However, there are discrepancies in the consistent specificity and sensitivity of the panel which limits the reliability of the test.¹⁷

Therefore, the identification of the MSI by the extension method described herein would decrease the time of diagnosis and increase sensitivity. The screening of multiple MSI markers from one DNA sample simultaneously would be achievable when used in an array. The BAT25 mononucleotide repeat sequence was used to test the efficiency of this method for microsatellite instability detection. The probe and target strands used to form the BAT25 oligo seed are displayed in Table 3. The amino-modified duplex extension was trialled in solution before attempting the extension off the surface.

**Table 3. The amino-modified DNA sequences used for the BAT25 extension**

| Name | Sequence (X = amino-c6) |
|---|---|
| BAT25-probe | 5' - X - GAAAAAAAAAACGAAAAAAAAAAC - 3' (SEQ ID NO: 17) |
| BAT25-target | 5' - GTTTTTTTTTTCGTTTTTTTTTTC - 3' (SEQ ID NO: 18) |

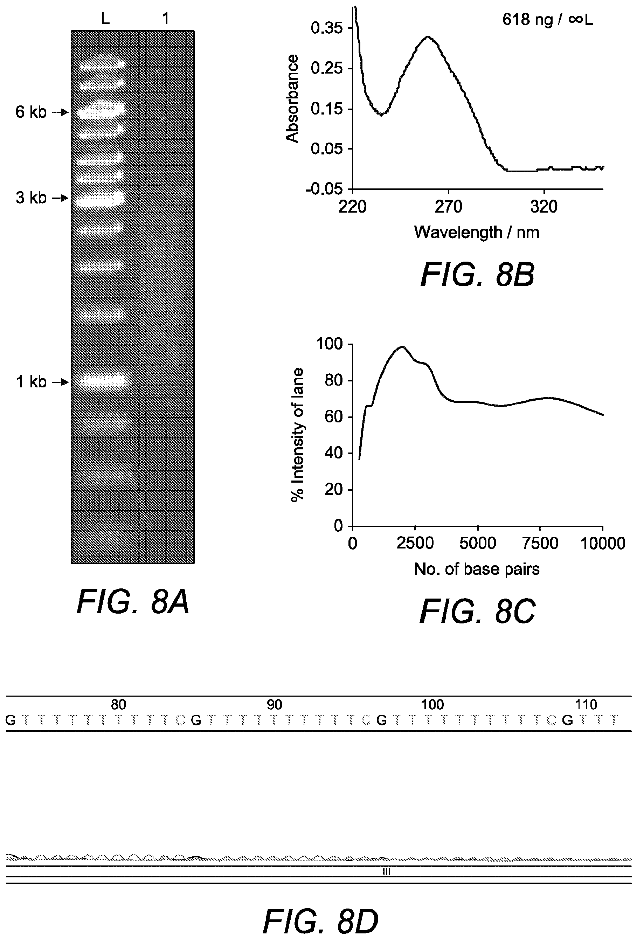

The BAT25 extension products were analysed by gel electrophoresis (see Figure 8a) and possessed a modal length of 2000 bp. DNA Sanger sequencing was carried out by GATC-biotech on a 5-cycle extension product for BAT25 sequence (see Figure 8d). The sequencing results confirmed the accurate incorporation of each base by the DNA polymerase. DNA sequencing was not attainable for the GATC and CFTR sequence, without wishing to be bound to any particular theory, this is possibly due to the challenges associated with GC-rich DNA sequences. Both the GATC and CFTR sequences contain 50 % GC content.

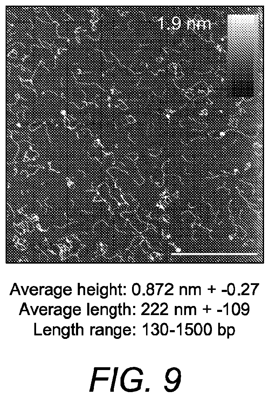

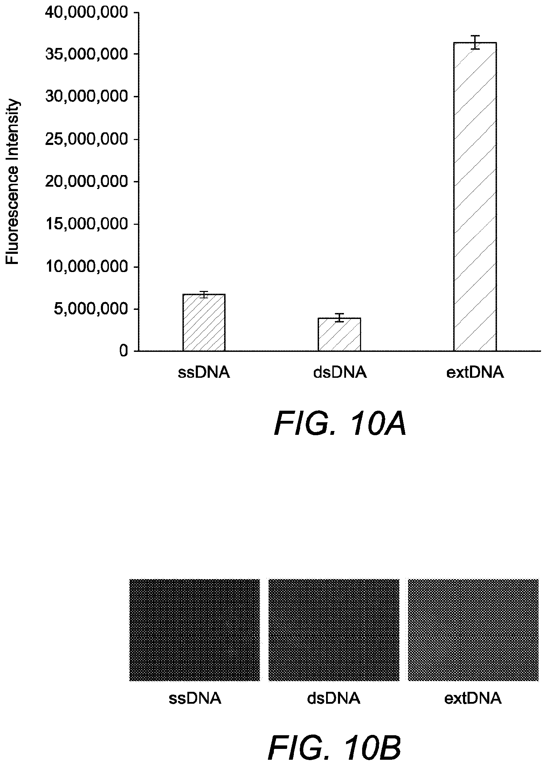

The DNA length for the BAT25 sequence was longer compared to the GATC and CFTR sequences with a higher concentration of 618 ng/µL indicating improved extension. The bp length ranged from approximately 750 bp to 3000 bp also confirmed by AFM analysis of the dsDNA extension products (Figure 9). The average height of the dsDNA was calculated to be 0.87 nm with the DNA length ranging from 130 to 1500, consistent with the gel analysis. The BAT25 sequence was extended off the surface after attachment of the amino-modified oligo seed. Addition of PG revealed increased fluorescence for the extDNA compared to the short-immobilised oligo seeds (see Figure 10).

The same increase in contact angle measurement was observed for the BAT25 extDNA sequence as for the CFTR sequence.

**Table 4. Contact angles of a water droplet on the ssDNA, dsDNA and extDNA surface for the BAT25 sequence.**

| Sample | Contact Angle /° |
|---|---|
| ssDNA | 29.64 |
| dsDNA | 25.58 |
| extDNA | 74.84 |

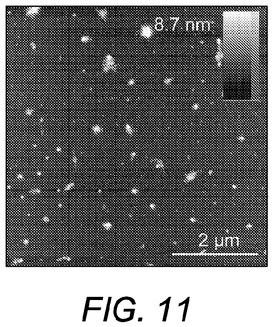

The double-stranded extended DNA (dsextDNA) strands were dehybridised as described under 8.5 below and combed onto freshly cleaved mica for AFM analysis (see Figure 11). Due to the susceptibility of single stranded AT rich DNA to fold and stack,^{18,19} an abundance of the strands appeared aggregated however, several strands were elongated and available for height and length analysis. The height of the single strands on average was 0.5 nm with an average length of 70 nm corresponding to 200 bp.

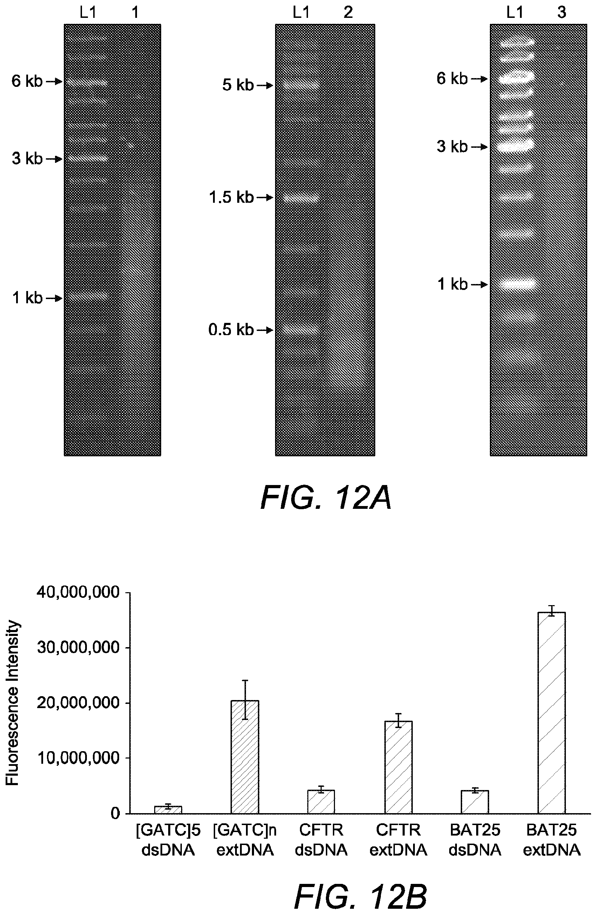

The enhanced fluorescence intensity for the BAT25 sequence was greater than observed in both the GATC and the CFTR sequences. A comparison of the gel electrophoresis and fluorescence intensity for the GATC, CFTR and BAT25 sequences (see Figure 12), confirmed the longer extension both in solution and off the surface.

The extension of the BAT25 sequence exhibited the greatest increase in fluorescence intensity compared to the other sequences studied and has medical importance, therefore the BAT25 sequences was used for further investigations into the sensing applications of the device.

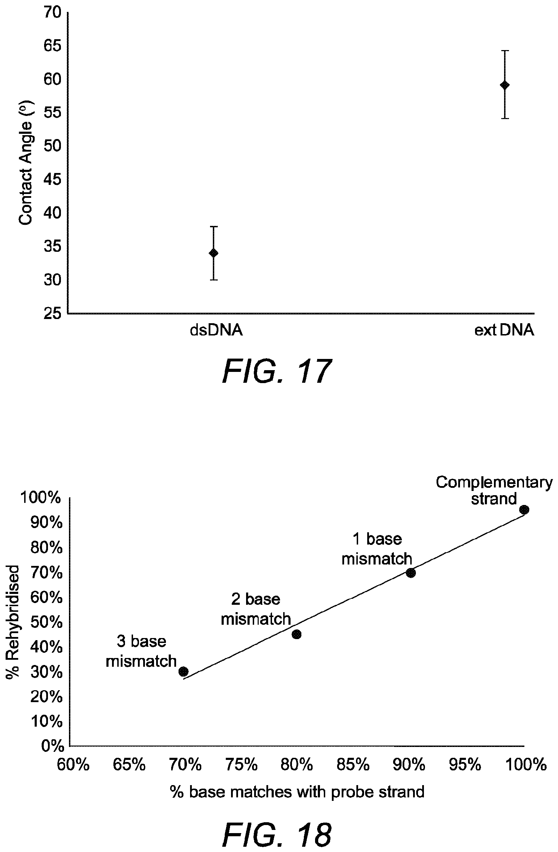

**Table 5. Mismatched sequences to be rehybridized with BAT25 probe (for figure 18)**

| | |
|---|---|
| Single base mismatch | 5-'GTTTTTTTTTTC-3' (SEQ ID NO:16) |
| Double base mismatch | 5'-GTTTTTTTTTC-3' (SEQ ID NO: 25) |
| Triple base mismatch | 5'-GTTTTTTTTC-3' (SEQ ID NO:26) |

### 8. Optimisation of parameters

Despite the distinguished difference in fluorescence intensity for the extDNA compared to the short oligo seed, several parameters to improve the sensitivity and reliability were carried out.

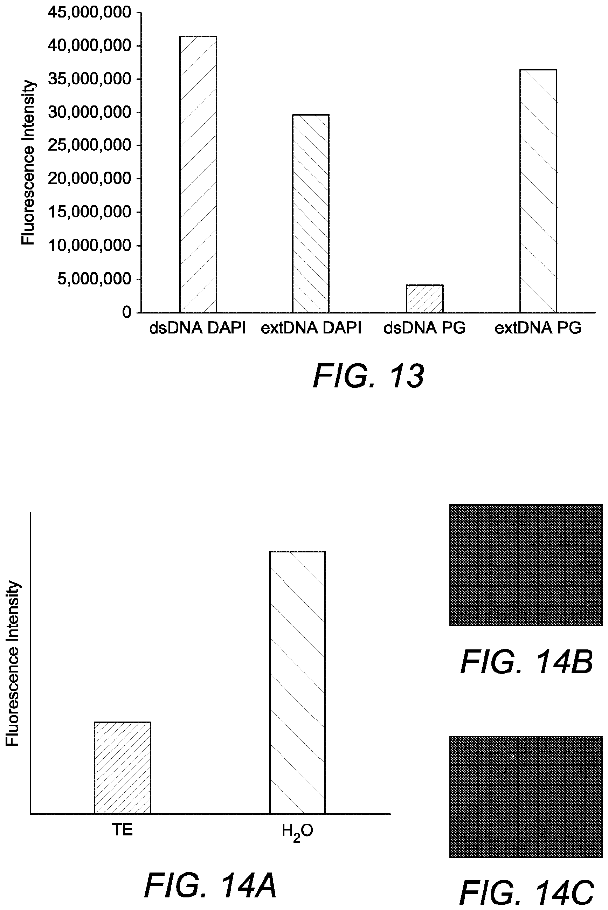

PG is established as a dsDNA intercalator irrespective of the base pairs. Other potential fluorescent dyes which are sequence specific could further enhance the fluorescence intensity change for extDNA samples. The BAT25 sequence is AT-rich therefore the fluorescent stain DAPI was analysed for suitability. DAPI is a popular stain due to the 20x increase in fluorescence upon binding to AT regions in dsDNA.²⁰ DAPI solution was placed on the surface for 20 min and fluorescent images were obtained with excitation at 360 nm and the difference in fluorescence intensity was observed (Figure 13). The fluorescence intensity (FI) for the extDNA sample was lower than for the short dsDNA. The difference between dsDNA and extDNA using PG was consistently observed to a greater degree, therefore PG continued to be used.

PG is traditionally used in a Tris-EDTA buffer, TE,²¹ however when placing the PG-TE solution onto the surface, several salt spots were visible which were not seen if PG was deposited in nanopure H₂O (see Figure 14b). The FI for PG in H₂O was also significantly higher than when PG was in TE therefore for future studies PG was dissolved in H₂O.

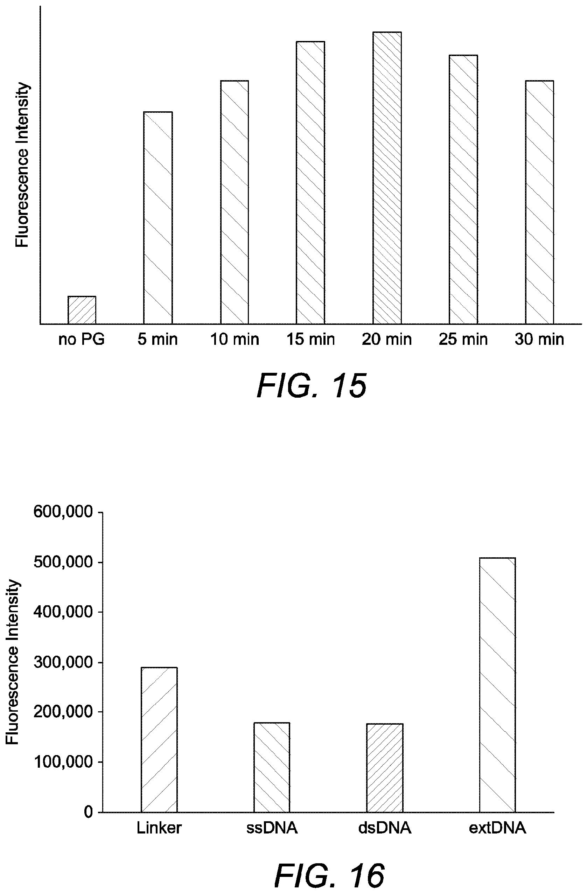

Many papers state the binding time of PG is almost instantaneous upon interaction with DNA,²¹ however with the DNA localised onto the surface, it could take longer for the intercalation of PG into the DNA to take place. PG was placed on extDNA surfaces for varying times and the fluorescence intensities compared (see Figure 15). After consecutive increase in FI with 5 min time increments, the FI decreased after 20 min. Therefore, for subsequent investigations the PG was placed on the surface for 20 min before washing.

All extensions from the surface were initially carried out on cut glass microscope slides. In order to see if the same phenomenon is observed on a different surface the extension protocol was performed on a silicon wafer. The extension method was carried out on silicon wafers, p-type (100) cut to 0.5 cm² and PG was applied to the surface for fluorescence imaging (see Figure 16). An increase in fluorescence was observed from short dsDNA to extDNA however the difference was smaller than as seen on a glass surface. Nevertheless, there was still a clear enhancement for the extDNA highlighting the versatility of the extension method. Not only the sequence but the surface can be tailored to the desired application.

### 9. Discussion of results

The methods described herein enable the synthesis of multiple probe sequences of DNA on a surface to enhance target detection. The invention capitalizes on the method of enzymatic synthesis of DNA (patent no GB17000531.5), which allows for the rapid synthesis of DNA with repeat units in solution. The repeat units can be tailored to match binding regions of molecules of interest such as DNA fragments indicative of a disease state. After denaturing (unwinding of the double stranded DNA) each single strand DNA has multiple binding sites in the vertical axis and so maximises the binding opportunities per surface bound probe strand. Binding with complementary target fragments results in increased fluorescence intensity compared to the short dsDNA on the surface. The extension increases the number of target binding sites per probe molecule hence increasing target detection and diagnostic sensitivity.

The inventors have demonstrated that the invention has excellent sensitivity thus addressing one of the key challenges in molecular diagnostics i.e. the detection of signal over the background noise. Due to the enhanced signal generation the sensitivity of the invention has exploitive routes in the detection of low numbers of target DNA molecules which in turn does not need the extended use of expensive and time-consuming sample preparation technologies such as PCR. Alternatively, the real cost benefit of this invention may lie in the enhanced data acquisition. This in turn means less sensitive detectors and less signal processing are required promoting the improved portability of diagnostic devices. A point of care device, which incorporates this technology, could be economically manufactured using cheaper parts, software and design elements, ultimately reducing the overall financial outlay. Point of care devices would benefit from such an enabling technology as presented here to become inexpensive in manufacture and appropriate for portable realisation.

### Experimental Materials and Methods

### Chemical reagents

All chemical reagents were purchased from Sigma Aldrich and used as received without further purification. Glass slides were purchased from Henso Labware Manufacturing Co., Ltd (Hangzhou, China). APEGDMES was purchased from NewChem Technologies Limited. DNA was purchased from Eurofins Genomics (Ebersberg, Germany). Tgo-Pol Z3 exo- was prepared and purified in house. (Jozwiakowski, S. K. & Connolly, B. A. A modified family-B archaeal DNA polymerase with reverse transcriptase activity. ChemBioChem 12, 35-37 (2011). Evans, S. J. et al. Improving dideoxynucleotide-triphosphate utilisation by the hyperthermophilic DNA polymerase from the archaeon Pyrococcus furiosis. Nucleic Acids Res 28, 1059-1066 (2000).)

### Primer template annealing

Primer duplexes for extension were prepared: DNA annealing buffer (10 mM Hepes pH 7.5, 100 mM NaCl and 1 mM EDTA) was added to the oligomers and heated to 95 °C for 10 min. Duplex solutions were slowly cooled to room temperature and stored at -20 °C.

### DNA polymerase

Tgo-Pol Z3 exo- DNA polymerase was used for the extension. The enzyme belongs to the Archael family B polymerase low fidelity variant with the 3'→5' exonuclease activity removed and alterations to the fingers domain.

### DNA extension in solution

0.5 µM DNA duplex, 200 nM Tgo-Pol Z3 exo- DNA polymerase, DNA polymerase reaction buffer (200 mM Tris-HCl (pH 8.8, 25 °C), 100 mM (NH₄)₂SO₄, 100 mM KCI, 1 % Triton X-100, 1 mg/mL Bovine Serum Albumin (BSA) and 20 mM MgSO₄), and 0.5 mM deoxynucleotide triphosphates (dNTPs) (dCTP, dATP, dTTP and dGTTP) were mixed together. Heat-cool thermocycles were performed on an Applied Bioscience Veritt 96 well Thermal Cycler for the following cycles:
Number of cycles (20) × 30 seconds at 95 °C, 30 seconds at 55 °C and 2 min at 72 °C. The solution was cooled to 4 °C after the reaction. The DNA extension product was purified using QIAquick PCR purification kit (25) (QIAGEN, Manchester, UK) following the manufacturers protocol.

### Agarose Gel Electrophoresis

DNA extension products were analysed by gel electrophoresis in TBE buffer (Tris, Boric acid and Na₂EDTA.2H₂O). 1 % Agarose (Melford, Ipswich, UK) was added to 1% TBE buffer and heated until fully dissolved. The gel mixture was cooled to 50 °C and poured into a gel cassette to set. The DNA ladders, 1 kb and 1 kb+, (Thermo Scientific) were provided with a Loading dye (2.5 % Ficoll-400, 11 mM EDTA, 3.3 mM Tris-HCl (pH 8.0, 25 °C), 0.017 % SDS and 0.015 % bromophenol blue). 2 µL gel loading dye was added to DNA samples (20 ng/µL) and loaded into the gel wells. The gels were run at 100 V, 100 mA 10 W for approximately 1 hr. The gel was post-stained with 5 µg/mL solution of ethidium bromide and visulaised using an UV transilluminator.

### UV

UV-Vis spectroscopy was performed using a Nanodrop. The spectrometer was blanked using nanopure H₂O.

### Pretreatment of surface

Glass slides or n-type Si<111> wafers were diced into 0.5 cm² chips. The chips were wiped with acetone, IPA and NP-H₂O and sonicated in acetone, IPA and NP-H₂O for 15 mins sequentially and dried with N₂. The chips were placed under O₂ plasma treatment for 15 min.

### Oligoseed DNA attachment via APEGDMES linker

The precleaned chips were immersed in APEGDMES/toluene solution (233 µM, 3 mL) preheated to 65 °C for 16 h. The chips were washed with toluene, ethanol and NP-H₂O 3 times sequentially before being placed in a vacuum oven at 120 °C for 40 min. Amino-tagged DNA probe solution (40 µL, 100µM) in 10% acetic acid solution was drop cast onto the chips for 1 h in a humid environment. NaCNBH₃ (40 µL, 16 µM) in 50 % MeOH solution was deposited on top of the probe solution for a further 2 h in a humid environment. The chips were washed with phosphate buffered saline (0.5 x) and excess water to remove any excess DNA molecules. Chips were dried with a stream of nitrogen.

### DNA hybridisation

Complementary DNA target solution (40 µL, 200 nM) in PBS buffer (0.5 x) was drop cast onto the silicon chips for 15 min in a humid environment. Chips were washed with PBS buffer (0.5 x) for 30 min and NP-H₂0 for 30 min then dried under a stream of nitrogen.

### DNA extension off the surface

The chips were placed in an Eppendorf along with the required solution for heat-cool cycles: 200 nM DNA polymerase, DNA polymerase reaction buffer (200 mM Tris-HCl (pH 8.8, 25 °C), 100 mM (NH₄)₂SO₄, 100 mM KCI, 1 % Triton X-100, 1 mg/mL Bovine Serum Albumin (BSA) and 20 mM MgSO₄), and 0.5 mM deoxynucleotide triphosphates (dNTPs) (dCTP, dATP, dTTP and dGTTP). Heat-cool thermocycles were performed on an Applied Bioscience Veritt 96 well Thermal Cycler for the following cycles:
Number of cycles (20) × 30 seconds at 95 °C, 30 seconds at 55 °C and 2 min at 72 °C.

The solution was cooled to 4 °C after the reaction. The chips were removed from the solution and washed in NP-H₂O for 30 min and dried under a stream of nitrogen.

### Oligoseed DNA attachment via biotin-streptavidin-biotin linker

Glass surfaces were cleaned using piranha solution followed by washing with H₂O and drying. A solution of 2-carbomethoxyethyltrichlorosilane was added in dry toluene in < 25 % humidity. After 1 hour, the glass was washed with acetone, ethanol, then H₂O, then filled with HCl and left over night. The glass was washed thoroughly with H₂O and covered with 50 mM 1-ethyl-3-(3-dimethylaminopropyl)carbodiimide and 1 mM amine-PEG₂-biotin in 10 mM Hepes. After 1 hour, the glass was washed followed by pipetting 50 µL of 0.1 mg/mL streptavidin in 10 mM Tris, pH 7.9 onto the surface. After 1 hour, the glass was washed followed by patterning using a UV photo mask and UV irradiation. The surface was then washed with 10 mM Tris, pH 7.9 and 50 µL of 1 µM C₁₅-biotin in 10 mM Tris, pH 7.9 and 200 mM NaCl was added to the same spot. After 1 hour, the surface was washed with 10 mM Tris-HCl and 50 µL 1 µM G₁₅ 10 mM Tris-HCl, pH 7.9 and 200 mM NaCl was added. After 1 hour, the glass surface was washed and filled with polymerisation solution; 0.5 mM dCTP and dGTP, 200 nM Tgo-Pol Z3 exo-, 200 mM NaCl, 0.5 mM MgCl₂ in 15 mM Tris HCl pH 7.6 and quenched by the removal of reaction solution and washing with 10 mM Tris, pH 7.9 and 200 mM NaCl. The surface was stained using SYBR Green.

### Fluorescence microscope imaging

Samples were placed on an Axioshop 2 plus (Zeiss, Germany) image platform, set to filter 44, with a Plan-NEOFLUAR 10 x/ 0.3 objective lens (Zeiss). The sample was excited at 490 nm from a ebq100 mercury lamp (LEJ, Germany) and images were taken using an AxioCam HRm (Zeiss).

### AFM

The top layer of the mica surface was cleaved using sticky tape. 5 µL of DNA sample (either 2 ng/µL or 4 ng/µL) was placed onto the mica surface held at a 25° angle to allow the DNA to flow across the mica surface. After 5 min, 5 µL nanopure H₂O was dropped on top of the DNA sample, again at 25° angle. A gentle stream of N₂ was passed over the surface and then further dried under laminar flow for 1 hr. AFM images were obtained using a Dimension V with a nanoscope controller (Veeco Instruments Inc., Metrology Group, Santa Barbara, CA) on an isolation table (Veeco Inc., Metrology Group) to reduce interference. The software NanoScope Analysis 1.8 was used to obtain data.

### Contact angle

Contact angle measurements were performed on a KSV Cam 101 (KSV Instruments Ltd., Finland) using built-in CAM 2008 software. A 1 µL drop of NP-H₂O was dropped onto the surface. The software was used to estimate the angle of the water droplet on the surface. 10 measurements for each sample were obtained. Measurements collected where there was more than a 2° difference in the left and right angle of the droplet were discarded. Measurements which were more than 2 standard deviations away from the average value were also discarded.

The reader's attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent, or similar purpose, unless expressly stated otherwise. Thus, unless expressly stated otherwise, each feature disclosed is one example only of a generic series of equivalent or similar features.

The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

### References

1 Gong, P. & Levicky, R. DNA surface hybridization regimes. Proceedings of the National Academy of Sciences 105, 5301-5306 (2008).
2 Metzker, M. L. Sequencing technologies - the next generation. Nature Reviews Genetics 11, 31 (2009).
3 Valignat, M.-P., Theodoly, O., Crocker, J. C., Russel, W. B. & Chaikin, P. M. Reversible self-assembly and directed assembly of DNA-linked micrometer-sized colloids. Proceedings of the National Academy of Sciences of the United States of America 102, 4225-4229 (2005).
4 Bracha, D., Karzbrun, E., Shemer, G., Pincus, P. A. & Bar-Ziv, R. H. Entropy-driven collective interactions in DNA brushes on a biochip. Proceedings of the National Academy of Sciences 110, 4534-4538 (2013).
5 Wang, C. et al. DNA microarray fabricated on poly(acrylic acid) brushes-coated porous silicon by in situ rolling circle amplification. Analyst 137, 4539-4545 (2012).
6 Jozwiakowski, S. K. & Connolly, B. A. A Modified Family-B Archaeal DNA Polymerase with Reverse Transcriptase Activity. ChemBioChem 12, 35-37 (2011).
7 Whitfield, C. J., Turley, A. T., Tuite, E. M., Connolly, B. A. & Pike, A. R. Enzymatic Method for the Synthesis of Long DNA Sequences with Multiple Repeat Units. Angewandte Chemie International Edition 54, 8971-8974, doi:10.1002/anie.201502971 (2015).
8 Terpilowski, K. & Rymuszka, D. Surface properties of glass plates activated by air, oxygen, nitrogen and argon plasma. Glass Physics and Chemistry 42, 535-541 (2016).
9 Dragan, A. I. et al. Characterization of PicoGreen Interaction with dsDNA and the Origin of Its Fluorescence Enhancement upon Binding. Biophysical Journal 99, 3010-3019 (2010).
10 Li, J. et al. A convenient method of aligning large DNA molecules on bare mica surfaces for atomic force microscopy. Nucleic Acids Research 26, 4785-4786 (1998).
11 Hansma, H. G., Sinsheimer, R. L., Li, M.-Q. & Hansma, P. K. Atomic force microscopy of single-and double-stranded DNA. Nucleic Acids Research 20, 3585-3590 (1992).
12 Hansma, H. G., Revenko, I., Kim, K. & Laney, D. E. Atomic Force Microscopy of Long and Short Double-Stranded, Single-Stranded and Triple-Stranded Nucleic Acids. Nucleic Acids Research 24, 713-720 (1996).
13 Mattheyses, A. L., Simon, S. M. & Rappoport, J. Z. Imaging with total internal reflection fluorescence microscopy for the cell biologist. Journal of Cell Science 123, 3621-3628 (2010).
14 R. A. Bartoszewski et al. A Synonymous Single Nucleotide Polymorphism in ΔF508 CFTR alters the Secondary Structure of the mRNA and the Expression of the Mutant Protein. J. Bio. Chem 285, 28741-28748 (2010).
15 Costa, D., Miguel, M. G. & Lindman, B. Responsive Polymer Gels: Double-Stranded versus Single-Stranded DNA. The Journal of Physical Chemistry B 111, 10886-10896 (2007).
16 Zhou, X.-P. et al. Determination of the replication error phenotype in human tumors without the requirement for matching normal DNA by analysis of mononucleotide repeat microsatellites. Genes, Chromosomes and Cancer 21, 101-107 (1998).
17 Umar, A. et al. Revised Bethesda Guidelines for Hereditary Nonpolyposis Colorectal Cancer (Lynch Syndrome) and Microsatellite Instability. Journal of the National Cancer Institute 96, 261-268 (2004).
18 Luzzati, V., Mathis, A., Masson, F. & Witz, J. Structure transitions observed in DNA and poly A in solution as a function of temperature and pH. Journal of Molecular Biology 10, 28-41 (1964).
19 Mills, J. B., Vacano, E. & Hagerman, P. J. Flexibility of single-stranded DNA: use of gapped duplex helices to determine the persistence lengths of Poly(dT) and Poly(dA)11Edited by B. Honig. Journal of Molecular Biology 285, 245-257 (1999).
20 Kapuscinski, J. DAPI: a DNA-Specific Fluorescent Probe. Biotechnic & Histochemistry 70, 220-233 (1995).
21 Singer, V. L., Jones, L. J., Yue, S. T. & Haugland, R. P. Characterization of PicoGreen Reagent and Development of a Fluorescence-Based Solution Assay for Double-Stranded DNA Quantitation. Analytical Biochemistry 249, 228-238 (1997).
22 Dodge, A., Turcatti, G., Lawrence, I., de Rooij, N. F. & Verpoorte, E. A Microfluidic Platform Using Molecular Beacon-Based Temperature Calibration for Thermal Dehybridization of Surface-Bound DNA. Analytical Chemistry 76, 1778-1787 (2004).
23 Lockett, M. R. & Smith, L. M. Fabrication and Characterization of DNA Arrays Prepared on Carbon-on-Metal Substrates. Analytical Chemistry 81, 6429-6437 (2009).
24 Eda, G., Fanchini, G. & Chhowalla, M. Large-area ultrathin films of reduced graphene oxide as a transparent and flexible electronic material. Nature Nanotechnology 3, 270 (2008).
25 Le, L. T., Ervin, M. H., Qiu, H., Fuchs, B. E. & Lee, W. Y. Graphene supercapacitor electrodes fabricated by inkjet printing and thermal reduction of graphene oxide. Electrochemistry Communications 13, 355-358 (2011).
26 He, Q. et al. Centimeter-Long and Large-Scale Micropatterns of Reduced Graphene Oxide Films: Fabrication and Sensing Applications. ACS Nano 4, 3201-3208 (2010).
27 Mohanty, N. & Berry, V. Graphene-Based Single-Bacterium Resolution Biodevice and DNA Transistor: Interfacing Graphene Derivatives with Nanoscale and Microscale Biocomponents. Nano Letters 8, 4469-4476 (2008).

## Claims

1. A thermocycling method for producing a microarray of DNA sequences, each comprising tandem repeats of a unit sequence that is 1 to 60 nucleotides long in a linear polynucleotide, the method comprising the steps of:
i) providing a solid substrate with a surface, the surface having immobilised thereon a plurality of distinct single stranded primer polynucleotides each comprising at least two tandem repeats of a unit sequence that is 1 to 60 nucleotides long,
ii) contacting the immobilised primer polynucleotides with single stranded template polynucleotides comprising at least two tandem repeats that are complementary to the unit sequence of the primer polynucleotides under hybridisation conditions that permit mismatched duplex formation between a unit sequence and its complement such that a 5' overhang of the template polynucleotide is generated, wherein the 5' overhang comprises at least one tandem repeat that is complementary to the unit sequence of the primer polynucleotide;
iii) contacting the mismatched duplexes with a thermostable 5' to 3' polymerase and nucleotides under extension conditions that permit polynucleotide extension in a 5' to 3' direction;
iv) denaturing the duplex of iii) under denaturing conditions to generate at least two different single stranded immobilised polynucleotides; and
v) repeating steps ii) to iii) to increase the number of tandem repeats in the immobilised polynucleotides.

2. The method of claim 1, wherein the solid substrate with a surface having immobilised thereon a plurality of different single stranded linear primer polynucleotides each comprising at least two tandem repeats of a unit sequence that is 1 to 60 nucleotides long, is provided by: immobilising at least two different double stranded linear primer polynucleotides each comprising at least two tandem repeats of a unit sequence that is 1 to 60 nucleotides long on the surface of the solid substrate; and denaturing the double stranded linear primer polynucleotides to provide the single stranded linear primer polynucleotides.

3. The method of claim 1 or 2, wherein the immobilised primer polynucleotide comprises at least 2, at least 5, at least 10, or at least 15 tandem repeats of the unit sequence, optionally wherein the unit sequence is a microsatellite sequence having 2 to 9 nucleotides or a minisatellite sequence having 10 to 60 nucleotides.

4. The method of any preceding claim, wherein the linear polynucleotides are immobilised to the surface by covalent or non-covalent bonding, optionally wherein the linear polynucleotides are non-covalently immobilised to a chemically modified region of the surface.

5. The method of any preceding claim, wherein the polynucleotides are immobilised to the surface by a linker.

6. The method of any preceding claim, wherein the linear polynucleotide is single stranded or double stranded DNA.

7. The method of any preceding claim, wherein the surface comprises glass, silica, gold, graphene or graphene oxide, epoxy, plastic, metal, gel matrix, template stripped metals or composites thereof.

8. The method of claim 7, wherein the linker comprises a silane linker molecule, a biotin-streptavidin complex, a thiol-Au linker, covalent Si-C bonds to silicon, covalent Si-O bonds to silicon, covalent Si-N bonds to silicon, a nanoparticle linker, or a dynamic covalent bond.

## Patentansprüche

1. Ein thermozyklisches Verfahren zur Herstellung eines Mikroarrays von DNA-Sequenzen, die jeweils Tandemwiederholungen einer Einzelsequenz mit einer Länge von 1 bis 60 Nukleotiden in einem linearen Polynukleotid umfassen, wobei das Verfahren die folgenden Verfahrensschritte umfasst:
i) die Bereitstellung eines festen Substrats mit einer Oberfläche, wobei auf der Oberfläche mehrere verschiedene einzelsträngige Primer-Polynukleotide fixiert sind, die jeweils mindestens zwei Tandemwiederholungen einer Einzelsequenz mit einer Länge von 1 bis 60 Nukleotiden umfassen,
ii) das Inkontaktbringen der fixierten Primer-Polynukleotide mit einzelsträngigen Matrizenpolynukleotiden, die mindestens zwei Tandemwiederholungen umfassen, die komplementär zu der Einheitensequenz der Primer-Polynukleotide sind, und dies unter Hybridisierungsbedingungen, die eine fehlangepasste Duplexbildung zwischen einer Einheitensequenz und ihrem Komplement ermöglichen, so dass ein 5'-Überhang des Matrizenpolynukleotids erzeugt wird, wobei der 5'-Überhang mindestens eine Tandemwiederholung umfasst, die komplementär zu der Einheitensequenz des Primer-Polynukleotids ist;
iii) das Inkontaktbringen der nicht übereinstimmenden Duplexe mit einer thermostabilen 5' zu 3' Polymerase und Nukleotiden unter Verlängerungsbedingungen, die eine Polynukleotidverlängerung in einer 5' zu 3' Richtung erlauben;
iv) die Denaturierung des Duplex aus iii) unter denaturierenden Bedingungen, um mindestens zwei verschiedene einzelsträngige fixierte Polynukleotide zu erzeugen; und
v) die Wiederholung der Verfahrensschritte ii) bis iii), um die Anzahl der Tandemwiederholungen in den fixierten Polynukleotiden zu erhöhen.

2. Das Verfahren nach Anspruch 1, wobei das feste Substrat mit einer Oberfläche, auf der eine Vielzahl verschiedener einzelsträngiger linearer Primer-Polynukleotide fixiert ist, die jeweils mindestens zwei Tandemwiederholungen einer Einheitensequenz umfassen, die 1 bis 60 Nukleotide lang ist, bereitgestellt wird durch: die Fixierung von mindestens zwei verschiedenen doppelsträngigen linearen Primer-Polynukleotiden, die jeweils mindestens zwei Tandemwiederholungen einer Einheitensequenz umfassen, die 1 bis 60 Nukleotide lang ist, auf der Oberfläche des festen Substrats; und die Denaturierung der doppelsträngigen linearen Primer-Polynukleotide, um die einzelsträngigen linearen Primer-Polynukleotide bereitzustellen.

3. Das Verfahren nach Anspruch 1 oder 2, wobei das fixierte Primer-Polynukleotid mindestens 2, mindestens 5, mindestens 10 oder mindestens 15 Tandemwiederholungen der Einheitensequenz umfasst, wobei es sich bei der Einheitensequenz gegebenenfalls um eine Mikrosatellitensequenz mit 2 bis 9 Nukleotiden oder eine Minisatellitensequenz mit 10 bis 60 Nukleotiden handelt.

4. Ein Verfahren nach einem der vorangegangenen Ansprüche, wobei die linearen Polynukleotide durch kovalente oder nicht kovalente Bindung an der Oberfläche fixiert sind, wobei die linearen Polynukleotide gegebenenfalls nicht kovalent an einer chemisch modifizierten Region der Oberfläche fixiert sind.

5. Das Verfahren nach einem der vorangegangenen Ansprüche, wobei die Polynukleotide durch einen Linker an der Oberfläche fixiert sind.

6. Das Verfahren nach einem der vorangegangenen Ansprüche, wobei das lineare Polynukleotid einzelsträngige oder doppelsträngige DNA ist.

7. Ein Verfahren nach einem der vorangegangenen Ansprüche, wobei die Oberfläche Glas, Siliziumdioxid, Gold, Graphen oder Graphenoxid, Epoxid, Kunststoff, Metall, Gelmatrix, Metalle erhalten nach dem Template-Stripping-Verfahren oder Verbundstoffe davon umfasst.

8. Das Verfahren nach Anspruch 7, wobei der Linker ein Silan-Linkermolekül, einen Biotin-Streptavidin-Komplex, einen Thiol-Au-Linker, kovalente Si-C-Bindungen an Silizium, kovalente Si-O-Bindungen an Silizium, kovalente Si-N-Bindungen an Silizium, einen Nanopartikel-Linker oder eine dynamische kovalente Bindung umfasst.

## Revendications

1. Procédé de thermocyclage pour la production d'un microréseau de séquences d'ADN, chacune comprenant des répétitions en tandem d'une séquence unitaire qui a une longueur de 1 à 60 nucléotides dans un polynucléotide linéaire, le procédé comprenant les étapes de :
i) fourniture d'un substrat solide avec une surface, la surface sur laquelle est immobilisée une pluralité de polynucléotides amorces simple brin comprenant chacun au moins deux répétitions en tandem d'une séquence unitaire qui a une longueur de 1 à 60 nucléotides,
ii) mise en contact des polynucléotides amorces immobilisés avec des polynucléotides matrices simple brin comprenant au moins deux répétitions en tandem qui sont complémentaires de la séquence unitaire des polynucléotides amorces dans des conditions d'hybridation qui permettent la formation de duplex mal appariés entre une séquence unitaire et son complément de telle sorte qu'un surplomb en 5' du polynucléotide matrice est généré, dans lequel le surplomb en 5' comprend au moins une répétition en tandem qui est complémentaire de la séquence unitaire du polynucléotide amorce ;
iii) mise en contact des duplex mal appariés avec une polymérase de 5' à 3' thermostable et des nucléotides dans des conditions d'extension qui permettent une extension du polynucléotide dans une direction 5' à 3' ;
iv) dénaturation du duplex de iii) dans des conditions dénaturantes pour générer au moins deux polynucléotides immobilisés simple brin différents ; et
v) répétition des étapes ii) à iii) pour augmenter le nombre de répétitions en tandem dans les polynucléotides immobilisés.

2. Procédé selon la revendication 1, dans lequel le substrat solide avec une surface sur laquelle est immobilisée une pluralité de polynucléotides amorces linéaires simple brin différents comprenant chacun au moins deux répétitions en tandem d'une séquence unitaire qui a une longueur de 1 à 60 nucléotides, est obtenu par : immobilisation d'au moins deux polynucléotides amorces linéaires double brin différents comprenant chacun au moins deux répétitions en tandem d'une séquence unitaire qui a une longueur de 1 à 60 nucléotides sur la surface du substrat solide ; et dénaturation des polynucléotides amorces linéaires double brin pour obtenir les polynucléotides amorces linéaires simple brin.

3. Procédé selon la revendication 1 ou 2, dans lequel le polynucléotide amorce immobilisé comprend au moins 2, au moins 5, au moins 10 ou au moins 15 répétitions en tandem de la séquence unitaire, éventuellement dans lequel la séquence unitaire est une séquence microsatellite ayant 2 à 9 nucléotides ou une séquence minisatellite comportant 10 à 60 nucléotides.

4. Procédé selon une quelconque revendication précédente, dans lequel les polynucléotides linéaires sont immobilisés sur la surface par liaison covalente ou non covalente, éventuellement dans lequel les polynucléotides linéaires sont immobilisés de manière non covalente dans une région chimiquement modifiée de la surface.

5. Procédé selon une quelconque revendication précédente, dans lequel les polynucléotides sont immobilisés sur la surface par un lieur.

6. Procédé selon une quelconque revendication précédente, dans lequel le polynucléotide linéaire est un ADN simple brin ou double brin.

7. Procédé selon une quelconque revendication précédente, dans lequel la surface comprend du verre, de la silice, de l'or, du graphène ou de l'oxyde de graphène, un époxy, du plastique, du métal, une matrice de gel, des métaux dénudés par gabarit ou des composites de ceux-ci.

8. Procédé selon la revendication 7, dans lequel le lieur comprend une molécule de lieur de silane, un complexe biotine-streptavidine, un lieur thiol-Au, des liaisons covalentes Si-C au silicium, des liaisons covalentes Si-Q au silicium, des liaisons covalentes Si-N au silicium, un lieur de nanoparticules ou une liaison covalente dynamique.
